(19) Europäisches Patentamt
European Patent Office
Office européen des brevets

(11) Veröffentlichungsnummer : **0 109 937**
**B1**

(12) # EUROPÄISCHE PATENTSCHRIFT

(45) Veröffentlichungstag der Patentschrift : **20.01.88**

(51) Int. Cl.⁴ : **G 03 C 7/26**// C07D401/12, C07D401/14

(21) Anmeldenummer : **83810530.2**

(22) Anmeldetag : **16.11.83**

(54) **Farbphotographisches Aufzeichnungsmaterial.**

(30) Priorität : **18.11.82 CH 6735/82**

(43) Veröffentlichungstag der Anmeldung : **30.05.84 Patentblatt 84/22**

(45) Bekanntmachung des Hinweises auf die Patenterteilung : **20.01.88 Patentblatt 88/03**

(84) Benannte Vertragsstaaten : **BE DE FR GB IT**

(56) Entgegenhaltungen :
**EP-A- 0 011 051**
**EP-A- 0 022 080**
**DE-B- 1 081 312**
**DE-C- 1 205 970**
**FR-A- 2 334 672**
**FR-A- 2 357 559**

(73) Patentinhaber : **CIBA-GEIGY AG**
**Klybeckstrasse 141**
**CH-4002 Basel (CH)**

(72) Erfinder : **Leppard, David G., Dr.**
**Route de Bourguillon 6**
**CH-1723 Marly (CH)**
Erfinder : **Rody, Jean, Dr.**
**Rütiring 82**
**CH-4125 Riehen (CH)**

**EP 0 109 937 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein farbphotographisches Aufzeichnungsmaterial, das in mindestens einer lichtempfindlichen Silberhalogenidemulsionsschicht und/oder in mindestens einer der üblichen Hilfsschichten als Stabilisator mindestens eine spezifische Polyalkylpiperidin-Verbindung enthält.

Polyalkylpiperidine sind als sterisch gehinderte Amine allgemein als Lichtschutzmittel für organische Materialien, insbesondere für Polymere, bekannt. Es wurde auch bereits in der DE-OS 2 126 954 vorgeschlagen, solche Polyalkylpiperidine als Mittel gegen das Ausbleichen von Farbphotographien zu verwenden. Es wurde weiterhin in der EP-A 11051 vorgeschlagen, als Lichtschutzmittel für Farbphotographien bestimmte Polyalkylpiperidinderivate zu verwenden, die mindestens eine Phenolgruppe enthalten. Es handelt sich dabei um Polyalkylpiperidinester von Hydroxylbenzylmalonsäuren.

In Weiterführung dieser Forschungsarbeiten wurde gefunden, dass s-Triazinderivate, die in ihrem Molekül mindestens eine sterisch gehinderte Phenolgruppe und mindestens eine Polyalkylpiperidingruppe enthalten, eine überraschend verbesserte Wirkung besitzen.

Gegenstand der vorliegenden Erfindung ist daher ein farbphotographisches Aufzeichnungsmaterial, das in mindestens einer lichtempfindlichen Silberhalogenidemulsionsschicht, einer Zwischenschicht, einer Bildempfangsschicht und/oder einer Schutzschicht als Stabilisator mindestens eine Verbindung der Formeln I, II oder III enthält,

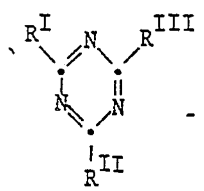 (I)

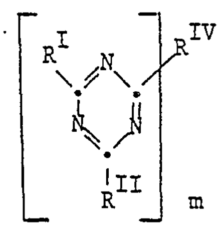 (II)

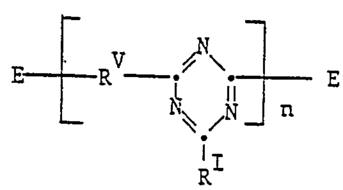 (III)

worin $R^I$, $R^{II}$ und $R^{III}$ unabhängig voneinander entweder

a) eine Gruppe der Formeln IV, V, VI oder VIa sind,

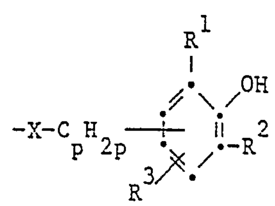 (IV)

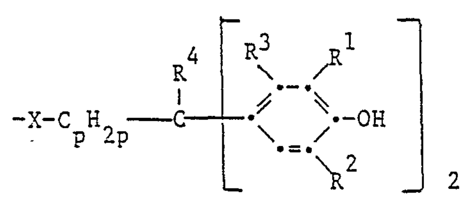 (V)

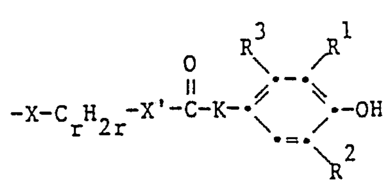 (VI)

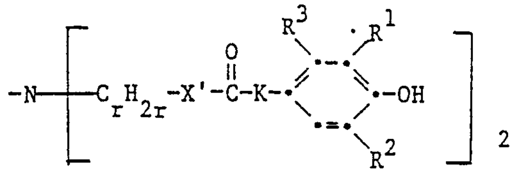

(VIa)

worin

p null oder eine Zahl von 1-12 ist

r eine ganze Zahl von 2-6 ist,

X —O—, —S— oder —$NR^5$— bedeutet,

X' —O— oder —$NR^5$— bedeutet

K eine direkte Bindung, $C_1$-$C_6$-Alkylen oder eine Gruppe der Formel

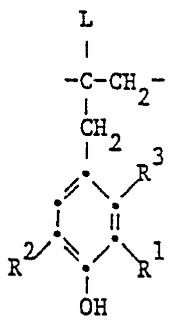

bedeutet,

$R^1$ Wasserstoff, $C_1$-$C_8$-Alkyl, $C_5$-$C_8$-Cycloalkyl, $C_7$-$C_9$-Phenylalkyl, Phenyl oder $C_7$-$C_{10}$-Alkylphenyl bedeutet,

$R^2$ $C_1$-$C_8$-Alkyl, $C_5$-$C_8$-Cycloalkyl, $C_7$-$C_9$-Phenylalkyl, Phenyl oder $C_7$-$C_{10}$-Alkylphenyl bedeutet,

$R^3$ Wasserstoff oder Methyl ist,

$R^4$ Wasserstoff, $C_1$-$C_{12}$-Alkyl, $C_5$-$C_8$-Cycloalkyl, Phenyl oder Benzyl bedeutet,

$R^5$ Wasserstoff, $C_1$-$C_{12}$-Alkyl, $C_5$-$C_{12}$-Cycloalkyl, Allyl oder Benzyl ist, und

L —CN, —$COCH_3$ oder —$SO_2CH_3$ bedeutet,

oder

b) eine Gruppe der Formeln VII bis XV sind,

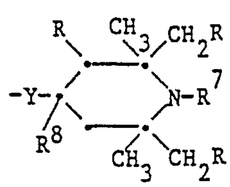

(VII)

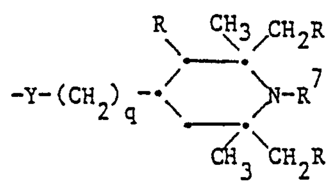

(VIII)

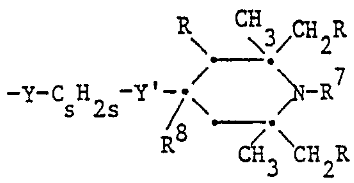

(IX)

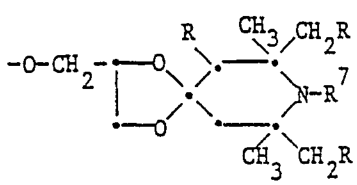

(X)

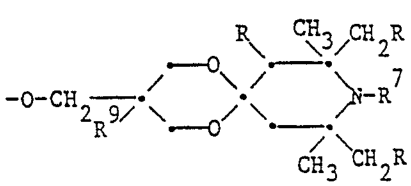

(XI)

(Fortsetzung)

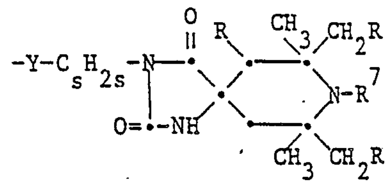
(XII)

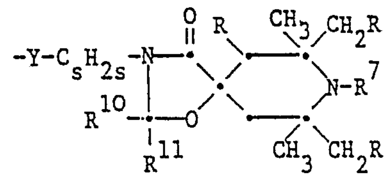
(XIII)

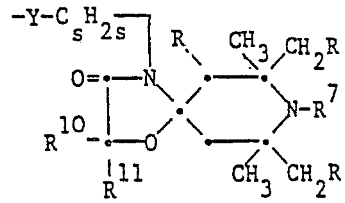
(XIV)

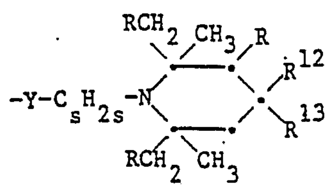
(XV)

worin

Y und Y' unabhängig voneinander —O— oder —N($R^6$)— bedeuten und

$R^6$ Wasserstoff, $C_1$-$C_{18}$-Alkyl, $C_3$-$C_6$-Alkenyl, $C_5$-$C_{12}$-Cycloalkyl, $C_7$-$C_{12}$-Phenylalkyl, $C_3$-$C_{12}$-Alkoxyalkyl, $C_4$-$C_{12}$-Dialkylaminoalkyl, eine Gruppe —$C_sH_{2s}$—X'—CO—$R^{14}$ oder

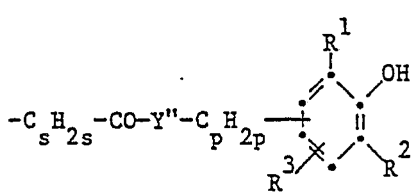

oder eine Gruppe der Formel XVI bedeutet,

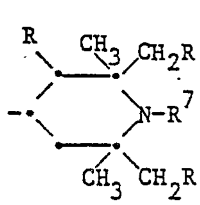
(XVI)

R Wasserstoff oder Methyl ist,

$R^7$ Hydroxyl, $C_1$-$C_{12}$-Alkyl, $C_3$-$C_6$-Alkenyl, $C_3$-$C_4$-Alkinyl, $C_7$-$C_{12}$-Phenylalkyl, Glycidyl, durch Halogen, —CN, —COOR$^{15}$ oder —CON($R^{16}$)($R^{17}$) substituiertes $C_1$-$C_4$-Alkyl, eine Gruppe —CO—$R^{14}$, —CO—OR$^{15}$, —CO—N($R^{16}$)($R^{17}$), —$CH_2$—CH($R^{18}$)—OR$^{19}$, —SO—$R^{20}$, —$SO_2$—$R^{20}$, —OR$^{15}$ oder —OOC—$R^{14}$ bedeutet,

$R^8$ Wasserstoff, —CN, —COOR$^{15}$, $CONH_2$ oder —CON($R^{16}$)($R^{17}$) bedeutet,

$R^9$ Methyl oder Ethyl ist,

$R^{10}$ Wasserstoff, $C_1$-$C_{12}$-Alkyl, $C_5$-$C_8$-Cycloalkyl oder Benzyl und

$R^{11}$ $C_1$-$C_{12}$-Alkyl, $C_5$-$C_8$-Cyloalkyl oder Phenyl ist, oder

$R^{10}$ und $R^{11}$ zusammen mit dem C-Atom, an das sie gebunden sind, einen $C_5$-$C_{12}$-Cycloalkan- oder Alkylcycloalkanring bilden,

$R^{12}$ Wasserstoff, $—OR^{21}$, $—OOC—R^{14}$, oder $—N(R^{22})—CO—R^{14}$ und

$R^{13}$ Wasserstoff, —CN, $—COOR^{15}$, $—CONH_2$ oder $—CON(R^{16})(R^{17})$ bedeutet oder

$R^{12}$ und $R^{13}$ zusammen eine Gruppe der Formeln

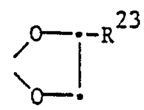
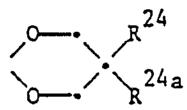
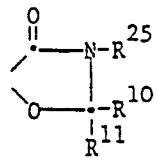

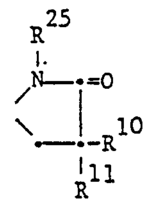
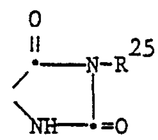

q 1 oder 2 ist,

s eine ganze Zahl von 2-6 ist,

Y" —O— oder $—NR^{5a}—$ bedeutet, worin $R^{5a}$ die Bedeutung von $R^5$ hat oder eine Gruppe der Formel XVI ist,

$R^{14}$ Wasserstoff, $C_1$-$C_{12}$-Alkyl, $C_2$-$C_6$-Alkenyl, Chlormethyl, $C_5$-$C_{12}$-Cycloalkyl, $C_7$-$C_{12}$-Phenylalkyl, Phenyl, $C_7$-$C_{10}$-Alkylphenyl oder durch 1 oder 2 $C_1$-$C_4$-Alkylgruppen und eine Hydroxygruppe substituiertes Phenyl, Phenylmethyl oder Phenylethyl bedeutet,

$R^{15}$ $C_1$-$C_{12}$-Alkyl, Allyl, Benzyl oder Cyclohexyl bedeutet,

$R^{16}$ $C_1$-$C_{12}$-Alkyl, Allyl, Cyclohexyl, Benzyl oder Phenyl, und

$R^{17}$ Wasserstoff, $C_1$-$C_8$-Alkyl oder Allyl bedeuten oder

$R^{16}$ und $R^{17}$ zusammen mit dem N-Atom einen 5- oder 6-gliedrigen heterocyclischen Ring bilden,

$R^{18}$ Wasserstoff, $C_1$-$C_4$-Alkyl, $C_2$-$C_{13}$-Alkoxymethyl, Phenyl oder Phenoxymethyl bedeutet,

$R^{19}$ Wasserstoff, $C_1$-$C_{12}$-Alkyl, $—CO—R^{14}$ oder $—CO—N(R^{16})(R^{17})$ bedeutet,

$R^{20}$ $C_1$-$C_{12}$-Alkyl, Phenyl oder $C_7$-$C_{22}$-Alkylaryl bedeutet,

$R^{21}$ Wasserstoff, $C_1$-$C_{12}$-Alkyl, Allyl oder Benzyl ist,

$R^{22}$ Wasserstoff, $C_1$-$C_{12}$-Alkyl, $C_5$-$C_{12}$-Cycloalkyl oder Benzyl ist,

$R^{23}$ Wasserstoff, Methyl oder Ethyl ist,

$R^{24}$ und $R^{24a}$ unabhängig voneinander H oder $C_1$-$C_4$-Alkyl sind und

$R^{25}$ Wasserstoff, $C_1$-$C_{12}$-Alkyl, $C_3$-$C_{12}$-Alkoxyalkyl, $C_5$-$C_{12}$-Cycloalkyl, Allyl oder Benzyl bedeutet, oder

c) eine Gruppe $—OR^{26}$, $—SR^{27}$, $—P(O)(OR^{28})_2$ oder $—N(R^{29})(R^{30})$ sind, worin

$R^{26}$ Wasserstoff, $C_1$-$C_{18}$-Alkyl, $C_3$-$C_6$-Alkenyl, $C_3$-$C_{12}$-Alkoxyalkyl, $C_4$-$C_{12}$-Dialkylaminoalkyl, Phenyl, $C_7$-$C_{22}$-Alkylaryl, $C_7$-$C_9$-Phenylalkyl oder $C_5$-$C_{12}$-Cycloalkyl bedeutet,

$R^{27}$ $C_1$-$C_{18}$-Alkyl, $C_5$-$C_{12}$-Cycloalkyl, Phenyl, $C_7$-$C_{12}$-Alkylphenyl, $C_7$-$C_9$-Phenylalkyl, $—CH_2COOR^{31}$ oder $—CH_2CH_2COOR^{31}$ und $R^{31}$ Wasserstoff oder $C_1$-$C_{12}$-Alkyl bedeutet,

$R^{28}$ $C_1$-$C_{12}$-Alkyl, Phenyl, Tolyl, Benzyl oder eine Gruppe der Formel XVI bedeutet,

$R^{29}$ und $R^{30}$ unabhängig voneinander Wasserstoff, $C_1$-$C_{12}$-Alkyl, durch $C_1$-$C_4$-Alkoxy, $R^{14}—COO—$ oder $C_2$-$C_8$-Dialkylamino substituiertes $C_2$-$C_4$-Alkyl, $C_5$-$C_{12}$-Cycloalkyl, $C_3$-$C_6$-Alkenyl oder $C_7$-$C_9$-Phenylalkyl bedeuten oder

$R^{29}$ und $R^{30}$ zusammen mit dem N-Atom, an das sie gebunden sind, einen 5- oder 6-gliedrigen heterocyclischen Ring bilden und

$R^{III}$ auch eine Gruppe der Formel XVII

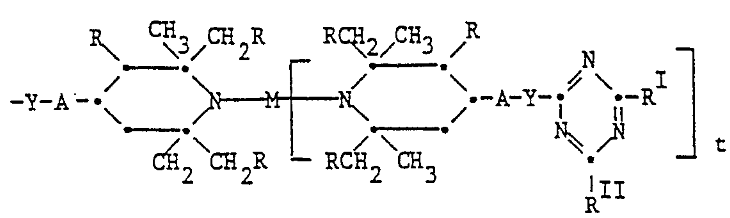

(XVII)

sein kann, worin t 1, 2 oder 3 ist,

A eine direkte Bindung, eine Gruppe $-(CH_2)_q-$ oder eine Gruppe $-Y'-C_sH_{2s}-$ ist und

M, wenn t = 1 ist, eine zweiwertige Gruppe ist und $C_2$-$C_{12}$-Alkylen, $C_4$-$C_8$-Alkenylen, Xylylen oder einen Rest der Formel

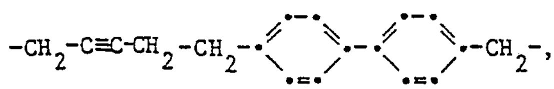, 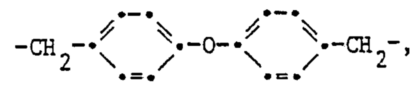,

$-CH_2-COO-B-OOC-CH_2-$, $-CH_2-CH(OH)-CH_2-$, $-CH_2CH(OH)CH_2-D-CH_2CH(OH)CH_2-$, $-CH_2-CH(R^{18})-OOC-R^{33}-COO-CH(R^{18})-CH_2-$ oder $-CO-NH-G-NH-CO-$

darstellt, worin B $C_2$-$C_{12}$-Alkylen, $C_4$-$C_8$-Oxaalkylen oder Cyclohexylen ist, D einen zweiwertigen Rest der Formel $-O-R^{32}-O-$ oder $-OOC-R^{33}-COO-$ darstellt, worin $R^{32}$ $C_2$-$C_{12}$-Alkylen, $C_6$-$C_{12}$-Cycloalkylen, $C_6$-$C_{12}$-Arylen oder -Phenyl-Z-Phenylen- und Z $-O-$, $-CH_2-$,

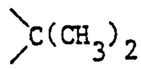

oder $-SO_2$ bedeutet,

$R^{33}$ eine direkte Bindung, $C_1$-$C_{12}$-Alkylen, $C_2$-$C_6$-Alkenylen, $C_6$-$C_{12}$-Cycloalkylen oder Cycloalkenylen oder $C_6$-$C_{12}$-Arylen bedeutet und G einen zweiwertigen aliphatischen, cycloaliphatischen, aromatischen oder aromatisch-aliphatischen Rest mit 6-15 C-Atomen bedeuten,

M, wenn t = 2 ist, einen dreiwertigen Rest der Formel

$T-[-CH_2CH(OH)CH_2-]_3-$ oder $R^{34}-[-COO-CH(R^{18})-CH_2-]_3-$

darstellt, worin T ein dreiwertiger Rest der Formeln

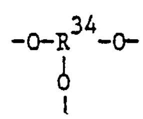 oder 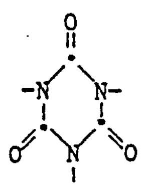 ist

und $R^{34}$ ein dreiwertiger aliphatischer Kohlenwasserstoffrest mit 3-10 Kohlenstoffatomen oder ein dreiwertiger aromatischer Kohlenwasserstoffrest mit 6-10 C-Atomen ist, und

M, wenn t = 3 ist, einen vierwertigen Rest der Formel

$Q-[-CH_2CH(OH)CH_2-]_4-$ oder $R^{35}-[-COO-CH(R^{18})-CH_2-]_4-$

darstellt, worin

Q eine Gruppe der Formel

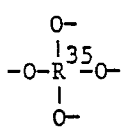 oder 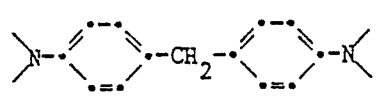

darstellt, und $R^{35}$ einen vierwertigen aliphatischen Kohlenwassersotffrest mit 4-10 C-Atomen oder einen vierwertigen aromatischen Kohlenwasserstoffrest mit 6-12 C-Atomen bedeutet,

$R^{IV}$ der m-wertige Rest eines Polyamines oder eines Polyols ist, und

m eine ganze Zahl von 2-6 ist,

$R^V$ eine zweiwertige Gruppe $-Y-R^{36}-Y-$ oder

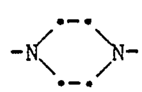

ist, worin

$R^{36}$ $C_2$-$C_{12}$-Alkylen, $C_5$-$C_{12}$-Cycloalkylen, $C_8$-$C_{12}$-Aralkylen, $C_6$-$C_{12}$-Arylen, durch O oder $NR^5$ unterbrochenes $C_4$-$C_8$-Alkylen oder eine Gruppe der Formel

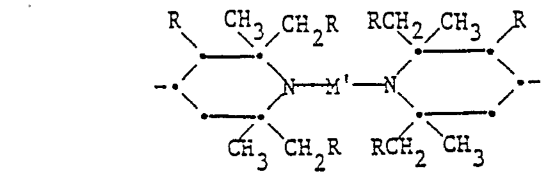

oder

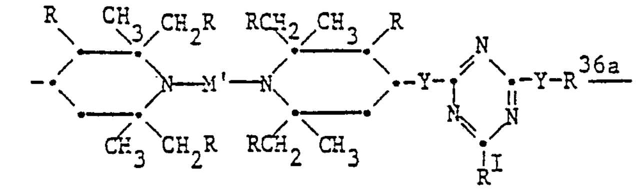

darstellt, worin

M' eine zweiwertige Gruppe der Formel M ist und

$R^{36a}$ $C_2$-$C_{12}$-Alkylen, $C_5$-$C_{12}$-Cycloalkylen, $C_8$-$C_{12}$-Aralkylen, $C_6$-$C_{12}$-Arylen oder durch O oder $NR^5$ unterbrochenes $C_4$-$C_8$-Alkylen bedeutet,

n 2 bis 50 ist und E und E' Endgruppen darstellen, wobei mindestens einer der Reste $R^I$, $R^{II}$, $R^{III}$ eine sterisch gehinderte Phenolgruppe enthält und mindestens einer der Reste $R^I$, $R^{II}$, $R^{III}$, $R^{IV}$, $R^V$ einen Polyalkylpiperidinrest enthält.

Wenn in diesen Definitionen $R^{18}$, $R^{24}$ oder $R^{24a}$ Alkylgruppen darstellen, so können diese z. B. Methyl, Ethyl, Isopropyl, n-Propyl, n-Butyl, Isobutyl oder tert.-Butyl sein. $R^1$, $R^2$ und $R^{17}$ können als $C_1$-$C_8$-Alkyl darüber hinaus auch z. B. n-Amyl, Isoamyl, n-Hexyl, 2-Ethylbutyl, iso-Heptyl oder n-Octyl sein. $R^4$, $R^5$, $R^7$, $R^{10}$, $R^{11}$, $R^{14}$, $R^{15}$, $R^{16}$, $R^{19}$, $R^{20}$, $R^{25}$, $R^{29}$ und $R^{31}$ können als $C_1$-$C_{12}$-Alkyl darüber hinaus auch z. B. n-Decyl, Isononyl oder n-Dodecyl sein. $R^6$, $R^{17}$ und $R^{26}$ als $C_1$-$C_{18}$-Alkyl können darüber hinaus auch z. B. n-Tetradecyl, n-Hexadecyl oder n-Octadecyl sein.

$R^6$, $R^{25}$ und $R^{26}$ als $C_3$-$C_{12}$-Alkoxyalkyl können z. B. 2-Methoxyethyl, 2-Ethoxyethyl, 2-Isopropoxyethyl, 3-Methoxypropyl, 2-Butoxyethyl, 2-Butoxypropyl oder 2-Hexaloxyethyl sein. $R^{18}$ als $C_2$-$C_{13}$-Alkoxymethyl kann z. B. Methoxy-, Ethoxy-, Butoxy-, 2-Ethylbutoxy- Hexyloxy- oder Dodecyloxymethyl sein.

$R^6$ und $R^{26}$ als $C_4$-$C_{12}$-Dialkylaminoalkyl können z. B. 2-Dimethylaminoethyl, 3-Diethylaminopropyl, 2-Dipropylaminoethyl oder 3-Dibutylaminopropyl sein.

$R^{29}$ und $R^{30}$ als durch $C_1$-$C_4$-Alkoxy, $R^{14}COO$— oder $C_2$-$C_8$-Dialkylamino substituiertes $C_2$-$C_4$-Alkyl können z. B. 2-Methoxyethyl, 2-Butoxyethyl, 3-Ethoxypropyl, 2-Butoxypropyl, 2-Acetoxyethyl, 2-Benzoyloxypropyl, 3-Lauroyloxypropyl, 2-Propionyloxybutyl, 2-Dimethylaminoethyl, 3-Diethylaminopropyl oder 3-Dibutylaminopropyl sein.

$R^1$, $R^2$, $R^4$ und $R^{10}$ als $C_5$-$C_8$-Cycloalkyl können z. B. Cyclopentyl, Cyclohexyl, Methylcyclohexyl, Dimethylcyclohexyl, Cycloheptyl oder Cyclooctyl sein. $R^5$, $R^6$, $R^{14}$, $R^{22}$, $R^{25}$, $R^{26}$, $R^{27}$, $R^{29}$ und $R^{30}$ als $C_5$-$C_{12}$-Cyclohexyl können darüber hinaus auch z. B. Cyclodecyl oder Cyclododecyl sein.

$R^{14}$ als $C_2$-$C_6$-Alkenyl kann z. B. Vinyl, 2-Propenyl, Allyl, 2-Methylvinyl, 2,2-Dimethylvinyl, 3-Methylallyl oder 2-Butylvinyl sein. $R^6$, $R^7$, $R^{26}$ und $R^{29}$ als $C_3$-$C_6$-Alkenyl können z. B. Allyl, Methallyl, 3-Methylallyl oder 3-Dimethylallyl sein.

$R^7$ als $C_3$-$C_4$-Alkinyl kann z. B. Propargyl oder 3-Methylpropargyl sein.

Wenn $R^{10}$ und $R^{11}$ zusammen mit dem C-Atom, an das sie gebunden sind, einen Cycloalkan- oder Alkylcycloalkanring bilden, so kann dies z. B. ein Cyclopentan-, Cyclohexan-, Methylcyclohexan-, Dimethylcyclohexan-, Cycloheptan-, Cyclooctan- oder Cyclododecanring sein.

$R^1$, $R^2$, $R^{26}$ und $R^{27}$ als $C_7$-$C_9$-Phenylalkyl können z. B. Benzyl, 2-Phenylethyl, 3-Phenylpropyl oder 1-Phenylisopropyl sein. $R^6$, $R^7$, $R^{14}$, $R^{29}$ und $R^{30}$ als $C_7$-$C_{12}$-Phenylalkyl können darüber hinaus auch z. B. 3-Phenylbutyl oder 6-Phenylhexyl sein.

$R^1$, $R^2$ und $R^{14}$ als $C_7$-$C_{10}$-Alkylphenyl können z. B. Tolyl, Xylyl, Ethylphenyl, Isopropylphenyl oder tert-Butylphenyl sein. $R^{27}$ als $C_7$-$C_{12}$-Alkylphenyl kann darüber hinaus auch z. B. Isoamylphenyl oder n-Hexylphenyl sein. $R^{20}$ und $R^{26}$ als $C_7$-$C_{22}$-Alkylaryl können darüber hinaus auch z. B. Methylnaphthyl, Butylnaphthyl, Dibutylphenyl, Dioctylphenyl, Nonylphenyl oder Dodecylphenyl sein.

$R^{16}$ und $R^{17}$ sowie $R^{29}$ und $R^{30}$ können jeweils zusammen mit dem N-Atom, an das sie gebunden sind, einen 5- oder 6-gliedrigen heterocyclischen Ring bilden. Dies kann z. B. ein Pyrrolidin-, Piperidin-, Morpholin- oder 4-Methylpiperazinring sein.

B, M, $R^{32}$, $R^{36}$ und $R^{36a}$ als $C_2$-$C_{12}$-Alkylen können unverzweigtes Alkylen wie z. B. Di-, Tri-, Tetra-, Hexa-, Octa-, Deca- oder Dodecamethylen oder verzweigtes Alkylen wie z. B. 1,2-Propylen, 1,2-Butylen, 2,2-Dimethyl-1,3-propylen oder 2,5,5-Trimethylhexamethylen sein. $R^{33}$ als $C_1$-$C_{12}$-Alkylen kann darüber hinaus auch Methylen darstellen.

B als $C_4$-$C_8$-Oxaalkylen kann z. B. 3-Oxapentylen, 1,5-4-Oxaheptylen-2,6 oder 3,6-Dioxaoctylen-1,8 sein. $R^{36}$ und $R^{36a}$ als durch O oder $NR^5$ unterbrochenes $C_4$-$C_6$-Alkylen können darüber hinaus auch z. B. 3-Azapentylen-1,5, 4-Azaheptylen-2,6 oder 3-(Methylaza)-pentylen-1,5 sein.

M als $C_4$-$C_8$-Alkenylen kann z. B. 2-Butenylen-1,4, 3-Hexenylen-1,6 oder 4-Octenylen-1,8 sein. $R^{33}$ als $C_2$-$C_6$-Alkenylen kann z. B. Vinylen, Methylvinylen oder 2-Butenylen-1,4 sein.

$R^{32}$, $R^{33}$, $R^{36}$ und $R^{36a}$ als $C_6$-$C_{12}$-Cycloalkylen können z. B. 1,4-Cyclohexylen, 1,2-Cyclohexylen, 1,4-Endomethylencyclohexylen-1,2, 1,4-Dimethylencyclohexan, 4,4'-Dicyclohexylen oder 1,4-Decahydronaphthylen sein.

$R^{32}$, $R^{33}$, $R^{36}$ und $R^{36a}$ als $C_6$-$C_{12}$-Arylen können z. B. 1,2-Phenylen, 1,3-Phenylen, 1,4-Naphthylen oder 4,4'-Diphenylen sein. $R^{36}$ und $R^{36a}$ als $C_8$-$C_{12}$-Aralkylen können z. B. m-Xylylen, p-Xylylen oder 1,4-Dimethylen-naphthalin sein.

G als zweiwertiger aliphatischer, cycloaliphatischer, aromatischer oder aromatisch-aliphatischer Rest mit 6-15 C-Atomen kann z. B. Di-, Tetra-, Hexa-, Octa-, Dodecamethylen, 2,2,4-Trimethylhexamethylen, Cyclohexylen, 4,4'-Dicyclohexylenmethan, 1,4-Phenylen, 2,4-Tolylen, 1,4-Naphthylen oder 4,4'-Diphenylenmethan sein.

$R^{34}$ als dreiwertiger aliphatischer Rest mit 3-10 C-Atomen kann z. B. Propan-1,2,3-triyl, 1,1,1-Trimethylenethan oder 1,1,1-Trimethylenpropan sein.

$R^{34}$ als dreiwertiger aromatischer Rest kann z. B. Benzol-1,2,4-triyl oder Naphthalin-1,4,5-triyl sein.

$R^{35}$ als vierwertiger aliphatischer Rest mit 4-10 C-Atomen kann z. B. 1,2,3,4-Butantetrayl oder Tetramethylenmethan sein. $R^{35}$ als vierwertiger aromatischer Rest kann z. B. Benzol-1,2,4,5-tetrayl, Naphthalin-1,4,5,8-tetrayl oder Diphenyl-3,4,3',4'-tetrayl sein.

$R^{IV}$ kann der n-wertige Rest eines Polyamins sein, wie z. B. der zweiwertige Rest von 1,2-Diaminoethan, Tri-, Tetra-, Hexa-, Octa-, Deca- oder Dodecamethylendiamin, 4,4'-Diaminodiphenylmethan, Benzidin, 1,3-Diaminobenzol, 1,4-Diaminocyclohexan, 4,4'-Diaminodiphenyloxid oder 1,4-Di(aminomethyl)-cyclohexan ; der dreiwertige Rest von Diethyltriamin oder Dipropylentriamin ; der vierwertige Rest von Triethylentetramin oder von 1,5,8,12-Tetraazadodecan ; der fünfwertige Rest von Tetraethylenpentamin oder von 1,5,8,12,16-Pentaazahexadecan oder der sechswertige Rest von Pentaethylenhexamin. $R^{IV}$ als n-wertiger Rest eines Polyols kann z. B. der zweiwertige Rest von Ethylenglykol, 1,2-Propylenglykol, 1,4-Butandiol, 1,6-Hexandiol, 1,8-Octandiol, Diethylenglykol, Triethylenglykol, Hydrochinon, Brenzchatechin, 1,4-Xylylenglykol, N-Methyl-diethanolamin, Cyclohexandiol-1,4, 1,4-Di(hydroxymethyl)-cyclohexan oder 2,2-Diphenylolpropan oder der dreiwertige Rest von Glycerin, Trimethylolpropan, Trimethylolethan oder von Tris(4-hydroxyphenyl)-methan, der vierwertige Rest von Pentaerythrit oder von Erythrit, der fünfwertige Rest von Xylit oder Arabit oder der sechswertige Rest von Dipentaerythrit sein.

Bevorzugt sind als Stabilisatoren solche Verbindungen der Formel I, II oder III, welche in ihrem Molekül mindestens eine sterisch gehinderte Phenolgruppe und mindestens eine N-substituierte 2,2,6,6-Tetramethylpiperidingruppe enthalten.

Unter den Stabilisatoren der Formel I sind solche bevorzugt, worin $R^I$, $R^{II}$ und $R^{III}$

a) eine Gruppe der Formel IVa sind,

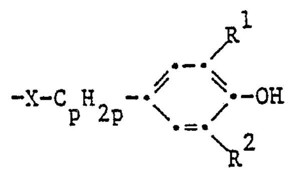

(IVa)

worin p eine ganze Zahl von 1 bis 4 ist und X, $R^1$ und $R^2$ die vorhin gegebene Bedeutung haben,

b) eine Gruppe der Formeln VIIa oder XVa sind,

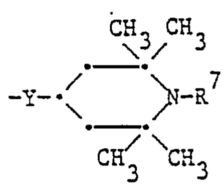

(VIIa),

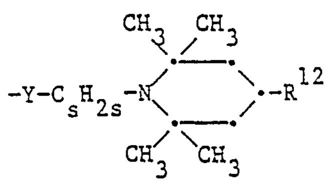

(XVa)

worin Y —O— oder —$NR^6$— bedeutet und $R^6$, $R^7$, $R^{12}$ und s die vorhin gegebene Bedeutung haben, oder

c) eine Gruppe —$OR^{26}$, —$SR^{27}$, —$P(O)(OR^{28})_2$ oder —$N(R^{29})(R^{30})$ sind, worin $R^{26}$, $R^{27}$, $R^{28}$, $R^{29}$ und $R^{30}$ die vorhin gegebene Bedeutung haben, wobei mindestens einer der Reste $R^I$, $R^{II}$, $R^{III}$ eine sterisch gehinderte Phenolgruppe enthält und mindestens einer der Reste $R^I$, $R^{II}$, $R^{III}$ eine N-substituierte 2,2,6,6-Tetremethylpiperidingruppe enthält.

Besonders bevorzugt sind solche Stabilisatoren der Formel I, worin $R^I$, $R^{II}$ und $R^{III}$ unabhängig voneinander entweder

a) eine Gruppe der Formel IVa sind, worin $R^1$ Wasserstoff oder $C_1$-$C_4$-Alkyl und $R^2$ $C_1$-$C_4$-Alkyl sind und X und p die vorhin gegebene Bedeutung haben, oder

b) eine Gruppe der Formel VIIa oder XVa sind, worin Y —O— oder —$NR^6$— bedeutet und $R^6$ Wasserstoff, $C_1$-$C_8$-Alkyl, Allyl, Cyclohexyl, Benzyl oder eine Gruppe —$C_sH_{2s}$—X'—CO—$R^{14}$ ist, worin s

= 2 oder 3 ist, X' —O— ist und $R^{14}$ durch 1 oder 2 $C_1$-$C_4$-Alkylgruppen und eine Hydroxylgruppe substituiertes Phenyl, Phenylmethyl oder Phenylethyl bedeutet, $R^7$ Methyl, Allyl, Benzyl, 2-Hydroxyethyl, Acetyl, Acryloyl oder eine Gruppe $—CON(R^{16})(R^{17})$ ist, worin $R^{16}$ $C_1$-$C_8$-Alkyl, Cyclohexyl oder Phenyl und $R^{17}$ Wasserstoff oder $C_1$-$C_8$-Alkyl bedeuten, und $R^{12}$ Wasserstoff ist, oder

c) eine Gruppe $—OR^{26}$, $—SR^{27}$ oder $—N(R^{29})(R^{30})$ sind, worin $R^{26}$ $C_1$-$C_{12}$-Alkyl, Phenyl, $C_7$-$C_{22}$-Alkylaryl, Benzyl oder Cyclohexyl bedeutet, $R^{27}$ $C_1$-$C_{12}$-Alkyl, Cyclohexyl, Phenyl, $—CH_2COO(C_1$-$C_{12}$-Alkyl) oder $—CH_2CH_2COO(C_1$-$C_{12}$-Alkyl) bedeutet und $R^{29}$ und $R^{30}$ Wasserstoff, $C_1$-$C_{12}$ Alkyl, Benzyl oder Allyl bedeuten.

Unter den Stabilisatoren der Formel II sind solche bevorzugt, worin m = 2 ist und $R^{IV}$ eine zweiwertige Gruppe der Formel

$$-N(R^6)-R^{37}-N(R^6)-$$ oder 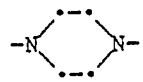

ist

worin $R^{37}$ $C_2$-$C_{12}$-Alkylen, durch —O— oder $—NR^5—$ unterbrochenes $C_4$-$C_8$-Alkylen, $C_6$-$C_{15}$-Arylen, $C_8$-$C_{12}$-Aralkylen oder $C_5$-$C_{12}$-Cycloalkylen ist und $R^5$ und $R^6$ die vorhin gegebene Bedeutung haben, sowie solche, worin m = 3 und $R^{IV}$ eine dreiwertige Gruppe der Formel

$$-N(R^6)-(CH_2)_a-\overset{|}{N}(R^6)-(CH_2)_a-N(R^6)-$$

ist oder m = 4 und $R^{IV}$ eine vierwertige Gruppe der Formel

$$-N(R^6)-(CH_2)_a-\overset{|}{N}(R^6)-(CH_2)_b-\overset{|}{N}(R^6)-(CH_2)_a-N(R^6)-$$

ist, worin a 2 oder 3 und b 2-12 bedeutet und $R^6$ die vorhin gegebene Bedeutung hat.

Unter den Stabilisatoren der Formel III sind solche bevorzugt, worin $R^V$ eine zweiwertige Gruppe $—N(R^6)—R^{36a}—N(R^6)—$ ist, in welcher $R^{36a}$ und $R^6$ die vorhin gegebene Bedeutung haben.

Besonders bevorzugt sind solche Stabilisatoren der Formel III, worin die Endgruppe E vorwiegend Wasserstoff ist und die Endgruppe E' vorwiegend eine Gruppe $—N(R^6)—R^{36a}—NHR^6$ ist.

Besonders bevorzugt sind weiterhin solche Stabilisatoren der Formel III, worin

$R^I$ eine Gruppe

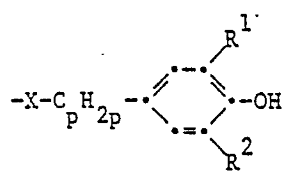

und

$R^V$ eine Gruppe

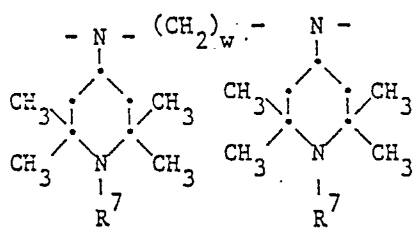

bedeutet, worin w 2-6 ist und $R^1$, $R^2$, X, p und $R^7$ die vorhin gegebene Bedeutung haben, insbesondere worin p 1-4 ist, X —O—, —S—, —NH— oder $—N(C_1$-$C_4$-Alkyl)— bedeutet, $R^1$ Wasserstoff oder $C_1$-$C_4$-Alkyl und $R^2$ $C_1$-$C_4$-Alkyl ist, w 2-6 ist und $R^7$ Methyl, Allyl, Benzyl, 2-Hydroxyethyl, Acetyl, Acryloyl oder eine Gruppe $—CON(R^{16})(R^{17})$ ist, worin $R^{16}$ $C_1$-$C_8$-Alkyl, Cyclohexyl oder Phenyl und $R^{17}$ Wasserstoff oder $C_1$-$C_8$-Alkyl bedeutet.

Die Verbindungen der Formel I sind aus dem US Patent 4 161 592 bekannt oder können in Analogie dazu durch stufenweise Reaktion von Cyanursäurechlorid mit den entsprechenden Verbindungen $R^IH$, $R^{II}H$ und $R^{III}H$ hergestellt werden.

Wenn $R^{III}$ eine Gruppe der Formel XVII ist, so können die Verbindungen aus den entsprechenden Verbindungen der Formel I, worin $R^{III}$ eine Gruppe

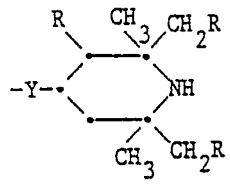

ist durch Umsetzung mit einem Dihalogenid, mit Epichlorhydrin oder mit einem Di-, Tri- oder Tetraepoxid gemäss US-Patent 4 294 963 oder durch Umsetzung mit einem Diisocyanat OCN—G—NCO gemäss Schweizer Patentanmeldung Nr. 9524/82 hergestellt werden.

Die Verbindungen der Formel II können in Analogie zum US Patent 4 108 829 durch stufenweise Reaktion von Cyanursäurechlorid mit den monofunktionellen Verbindungen $R^IH$ und $R^{II}H$ sowie mit einem Polyamin $R^{IV}(H)_m$ hergestellt werden.

Die Verbindungen der Formel III stellen Polymere oder Oligomere dar und können in Analogie zum US-Patent 4 086 204 durch stufenweise Umsetzung von Cyanursäurechlorid mit einer monofunktionellen Verbindung $R^IH$ und mit einer difunktionellen Verbindung $H—R^V—H$ hergestellt werden. Die Endgruppe E kann dabei H oder eine Gruppe

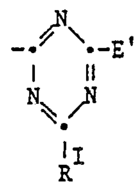

sein, die Endgruppe E' kann C1 oder eine Gruppe $—R^V—H$ sein.

Die Art der Endgruppen lässt sich durch Verwendung eines Ueberschusses der einen oder der anderen Polykondensations-Komponente so lenken, dass an beiden Molekülenden vorwiegend eine Endgruppe vorhanden ist. Ausserdem können die Endgruppen durch chemische Reaktionen modifiziert werden, wie das im US-Patent 4 234 707 beschrieben ist. Beispielsweise können durch Hydrolyse Chlortriazin-Endgruppen in Hydroxytriazin-Endgruppen umgewandelt werden.

Der Polykondensationsgrad n stellt ein Zahlenmittel dar, das die Werte 2 bis 50 annehmen kann. Dabei handelt es sich immer um ein Gemisch von Produkten verschiedenen Molekulargewichtes. Bevorzugt sind Produkte mit einem mittleren Polykondensationsgrad n = 2-10. Eine solche Begrenzung des Molekulargewichtes kann in an sich bekannter Weise dadurch erzielt werden, dass man eine der beiden Komponenten im Ueberschuss verwendet oder dass man eine monofunktionelle Verbindung als Kettenabbrecher zugibt.

Beispiele für einzelne Stabilisatoren der Formel I sind die Verbindungen der folgenden Formeln :

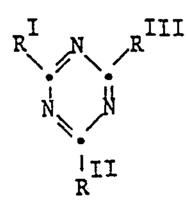

(1) $R^I = R^{II} = R^{III}$ = 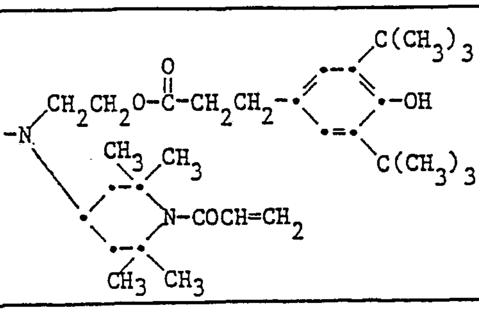

(2) $R^I = R^{II} = R^{III}$ = 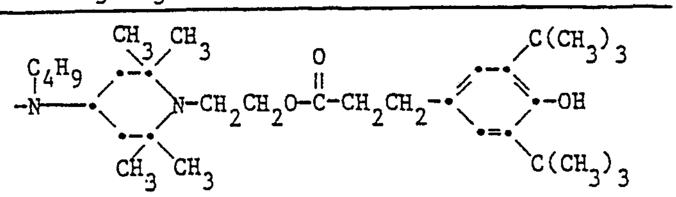

(Fortsetzung)

(3) $R^I$ =

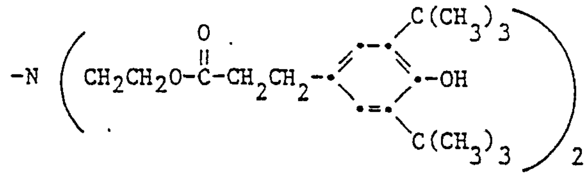

$R^{II}$ = $R^{III}$ =

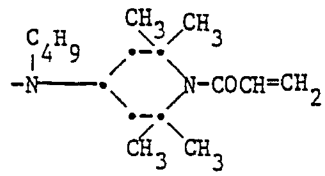

(4) $R^I$ =

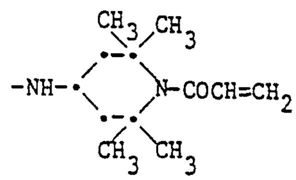

$R^{II}$ = $R^{III}$ =

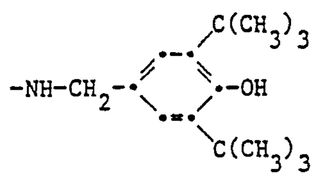

(5) $R^I$ =

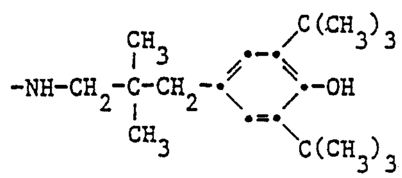

$R^{II}$ = $R^{III}$ =

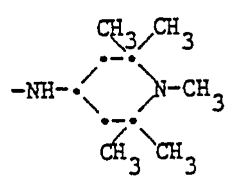

(6) $R^I$ = $-S-C_8H_{17}$

$R^{II}$ =

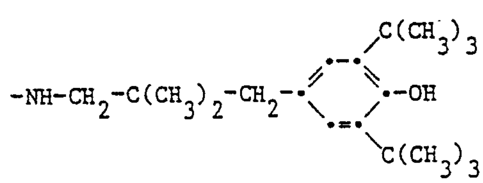

$R^{III}$ =

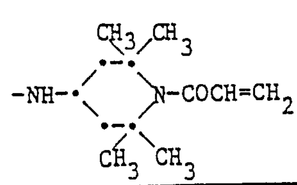

(Fortsetzung)

(7) $R^{I}$ = $-P(O)(OC_2H_5)_2$

$R^{II}$ = 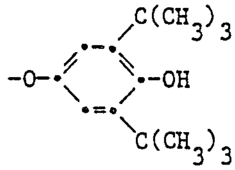

$R^{III}$ = 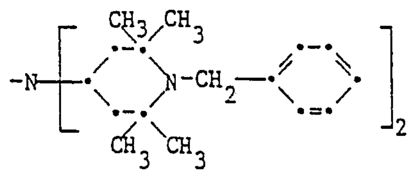

---

(8) $R' = R''$ = 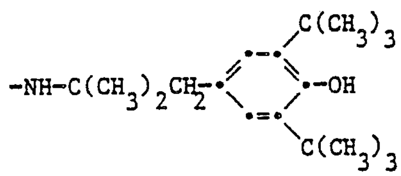

$R'''$ = 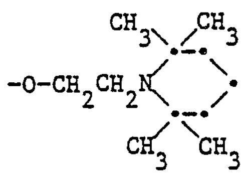

---

(9) $R'$ = 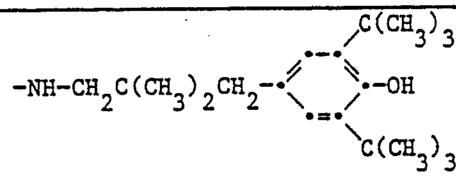

$R^{II} = R^{III}$ = 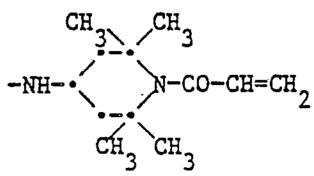

---

(10) $R'$ = $-NH-CH_2C(CH_3)_2CH_2C(CH_3)_3$

$R^{II} = R^{III}$ = 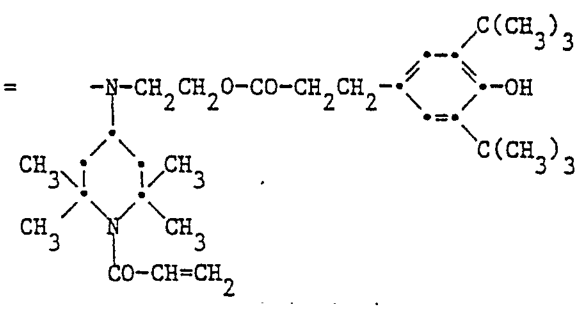

(Fortsetzung)

(11) R' = 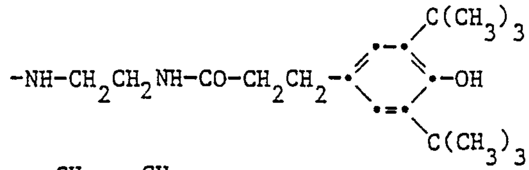

$R^{II} = R^{III}$ = 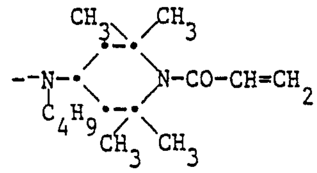

R' = 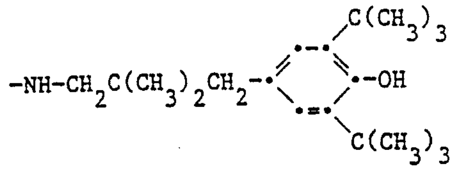

$R^{II} = R^{III}$ = 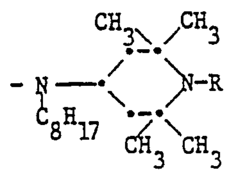

(12) R = $CO-CH=CH_2$

(13) R = 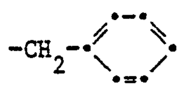

(14) $R^{I} = R^{II} = R^{III}$ = 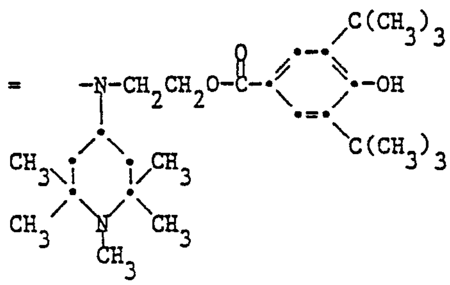

(15) $R^{I} = R^{II} = R^{III}$ = 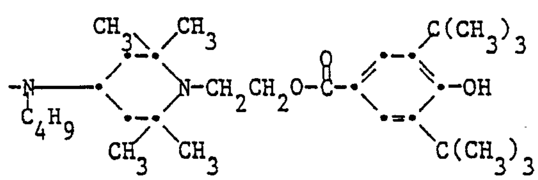

Beispiele für Stabilisatoren der Formel II sind folgende Verbindungen :

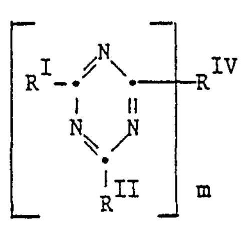

(Fortsetzung)

(16) $R^{I} = R^{II} =$ 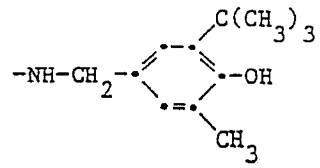

m = 2

$R^{IV} =$ 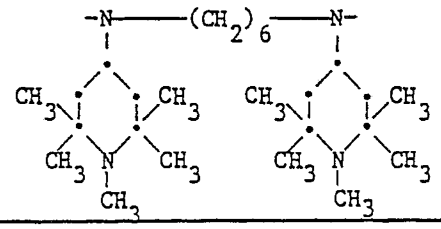

(17) $R^{I} =$ 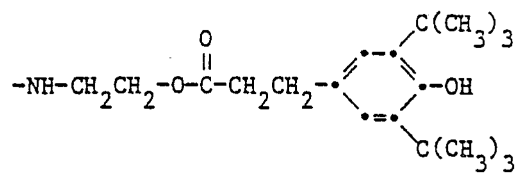

$R^{II} =$ 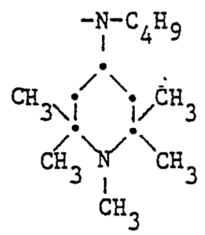

m = 3

$R^{IV} =$ $-NH-CH_2CH_2-\overset{|}{N}-CH_2CH_2-NH-$

(18) $R^{I} = R^{II} =$ 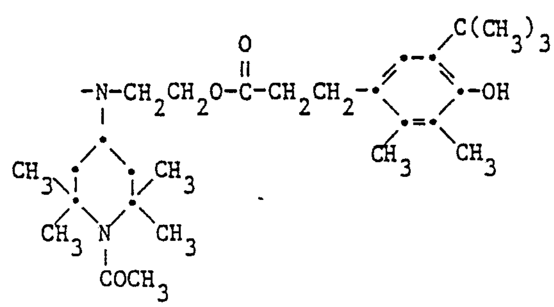

m = 4

$R^{IV} =$ $-NH-(CH_2)_3-\overset{|}{N}-(CH_2)_6-\overset{|}{N}-(CH_2)_3-NH-$

Beispiele für Verbindungen der Formel III sind folgende Verbindungen :

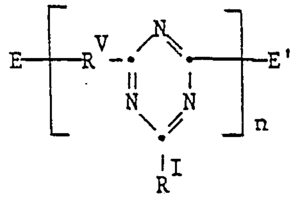

(Fortsetzung)

(19) $R^I$ =

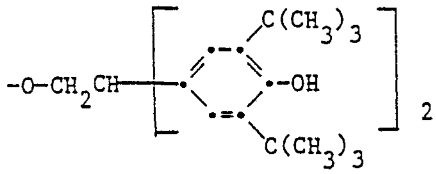

$R^V$ =

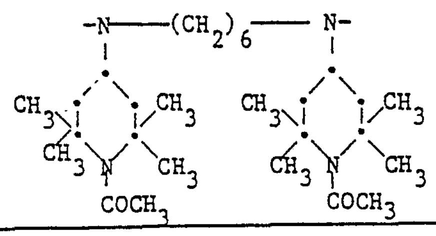

(20) $R^I$ =

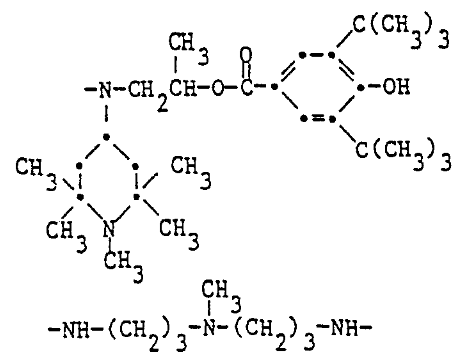

$R^V$ =

Die Stabilisatoren der Formel I, II und III können allein oder zusammen mit anderen Verbindungen in bekannter Weise in ein photographisches Material eingearbeitet werden.

In der Regel werden die Stabilisatoren allein oder zusammen mit anderen Verbindungen, insbesondere mit den Farbkupplern, in Form einer Dispersion in das photographische Material eingearbeitet, wobei diese Dispersion entweder kein Lösungsmittel oder hoch- oder tiefsiedende Lösungsmittel oder ein Gemisch solcher Lösungsmittel enthält. Eine weitere geeignete Einarbeitungsform besteht darin, dass man die Stabilisatoren allein oder zusammen mit weiteren Verbindungen zusammen mit einem Polymer in Form eines Latex in das photographische Material einarbeitet.

Die Dispersionen werden dann zur Herstellung der Schichten von farbphotographischen Aufzeichnungsmaterialien verwendet. Diese Schichten können z. B. Zwischen- oder Schutzschichten, insbesondere jedoch lichtempfindliche (blau-, grün und rot-empfindliche) Silberhalogenidemulsionsschichten sein, in denen bei der Entwicklung des belichteten Aufzeichnungsmaterials aus den entsprechenden Farbkupplern, die Blaugrün (Cyan)- Purpur (Magenta)- und Gelbfarbstoffe gebildet werden.

Die Silberhalogenidschichten können beliebige Farbkuppler, insbesondere Blaugrün-, Purpur- und Gelb-Kuppler, die zur Bildung der genannten Farbstoffe und damit der Farbbilder verwendet werden, enthalten.

Da das Substrat die Wirkung und Stabilität der Stabilisatoren beeinflusst, werden Substrate (Lösungsmittel, Polymere) bevorzugt, die zusammen mit den Stabilisatoren eine möglichst gute Beständigkeit der zu stabilisierenden Materialien ergeben.

In der Regel werden die Stabilisatoren in Schichten eingearbeitet, die zusätzlich eine nach üblichen Methoden hergestellte und sensibilisierte Silberhalogenid-Dispersion enthalten. Sie können jedoch auch in zu Silberhalogenid enthaltenden Schichten benachbarten Schichten vorhanden sein.

Die erfindungsgemässen photographischen Materialien besitzen einen üblichen Aufbau und Komponenten, die die Wirksamkeit der Stabilisatoren verstärken oder zumindest nicht nachteilig beeinflussen.

Im photographischen Aufzeichnungsmaterial gemäss vorliegender Erfindung können die Stabilisatoren der Formel I, II und III ausser mit den Farbkupplern zusätzlich auch mit Ultraviolettabsorbern oder anderen Lichtschutzmitteln in der gleichen Schicht kombiniert werden.

Wenn die Diffusionstransfermethode angewendet wird, kann der Stabilisator auch in eine Empfangsschicht eingearbeitet werden.

Die erfindungsgemässen farbphotographischen Materialien können in bekannter Weise verarbeitet werden. Ferner können sie im Verlauf oder nach der Verarbeitung in einer Weise behandelt werden, die

ihre Stabilität weiter erhöht, beispielsweise durch die Behandlung in einem Stabilisatorbad oder das Aufbringen eines Schutzüberzuges.

Die erfindungsgemäss einzusetzenden Stabilisatoren eignen sich in gewissen Fällen auch zum Schutz farbphotographischer Schichten, in denen die Farbstoffe direkt in die Emulsion eingelagert werden und das Bild durch selektive Bleichung erzeugt wird.

Die Menge des Stabilisators oder der Stabilisatoren kann in weiten Grenzen schwanken und liegt etwa im Bereich von 1 bis 2 000 mg, vorzugsweise 100 bis 800 und insbesondere 200-500 mg pro $m^2$ der Schicht, in die es (sie) eingearbeitet wird (werden).

Falls das photographische Material einen oder mehrere UV-Absorber enthält, so kann dieser mit dem Stabilisator zusammen in einer Schicht oder auch in einer benachbarten Schicht vorhanden sein. Die Menge an UV-Absorber kann in weiten Grenzen schwanken und liegt etwa im Bereich von 200-2 000 mg, vorzugsweise 400-1 000 mg pro $m^2$ der Schicht. Beispiele für geeignete UV-Absorber sind solche vom Benzophenon-, Acrylnitril-, Thiazolidon-, Benztriazol-, Oxazol-, Thiazol- und Imidazoltyp.

Die mit dem erfindungsgemässen Aufzeichnungsmaterial durch Belichtung und Entwicklung erhaltenen Farbbilder zeigen eine sehr gute Lichtechtheit gegenüber sichtbarem und ultraviolettem Licht. Die Verbindungen der Formel I, II und III sind praktisch farblos, so dass es zu keiner Verfärbung der Bilder kommt ; ausserdem sind sie gut verträglich mit den üblichen, in den einzelnen Schichten vorhandenen photographischen Zusatzstoffen. Aufgrund ihrer guten Wirksamkeit kann man ihre Einsatzmenge herabsetzen und vermeidet so ihre Ausfällung oder ihr Auskristallisieren, wenn man sie als organische Lösung in die wässerigen Bindemittelemulsionen, die für die Herstellung photographischer Schichten verwendet werden, einarbeitet. Die einzelnen nach der Belichtung des photographischen Aufzeichnungsmaterials notwendigen Verarbeitungsschritte zur Herstellung der Farbbilder werden durch die Stabilisatoren der Formel I, II und III nicht nachteilig beeinflusst. Ferner kann die bei blauempfindlichen Emulsionen häufig auftretende sogenannte Druckschleierbildung weitgehend zurückgedrängt werden. Diese kann z. B. auftreten, wenn auf photographische Materialien (Silberhalogenidemulsionsschichten, die auf einem Träger aus natürlichen oder synthetischen Materialien befinden) mechanische Beanspruchungen, z. B. Drehen, Biegen oder Reiben, während der Herstellung oder während der Behandlung vor der Entwicklung ausgeübt werden. (T. H. James, The Theory of Photographic Process 4. Auflage, Macmillan, New York, N. Y. 1977, Seite 23 ff., S. 166 ff.).

## Beispiel

0,087 g des Gelbkupplers der Formel

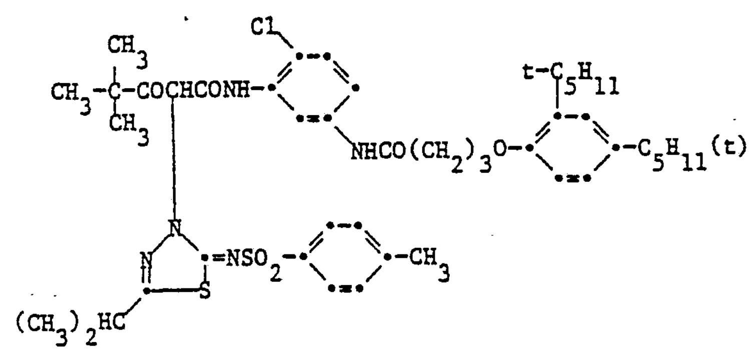

und 0,026 g eines der in der nachfolgenden Tabelle angegebenen Stabilisatoren werden in 2,0 ml eines Gemisches von Trikresylphosphat/Ethylacetat (1,5 g in 100 ml) gelöst. Zu dieser Lösung gibt man 7,0 ml einer 6 %igen Gelatinelösung, 0,5 ml einer 8 %igen Lösung des Netzmittels der Formel

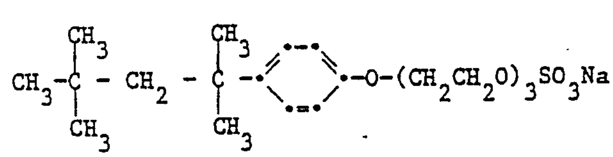

in Isopropanol/Wasser (3 : 4) und 0,5 ml Wasser und emulgiert mit Ultraschall bei einer Leistung von 100 Watt während 5 Minuten.

Zu 2,5 ml der so erhaltenen Emulsion gibt man 2,0 ml einer Silberbromid-Emulsion mit einem Silbergehalt von 6,0 g pro Liter, 0,7 ml einer 1 %igen wässerigen Lösung des Härters der Formel

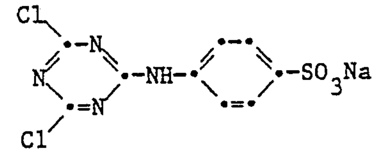

und 3,8 ml Wasser, stellt das Gemisch auf einen pH-Wert von 6,5 und vergiesst es auf ein auf eine Glasplatte aufgezogenes substriertes, kunststoffbeschichtetes, weisses Papier.

Nach dem Erstarren wird in einem Trockenschrank mit Umluft bei Raumtemperatur getrocknet.

Nach 7 Tagen werden auf 35 × 180 mm geschnittene Proben hinter einem Stufenkeil mit 3 000 Lux·s belichtet und anschliessend im Ektaprint® 2-Prozess der Firma Kodak verarbeitet.

Die so erhaltenen Gelbkeile werden in einem Atlas Weather-Ometer mit einer 2 500 W-Xenonlampe mit total 42 kJoule/cm$^2$ bestrahlt (eine Vergleichsprobe enthält kein Lichtschutzmittel). Der dabei eingetretene Farbdichteverlust wird bestimmt durch Messung der Farbdichte bei $\lambda_{max}$ mit einem Densitometer (TR 924 A der Fa. Macbeth).

Die Ergebnisse sind in der nachfolgenden Tabelle aufgeführt.

| Stabilisator Verbindung Nr. | Prozentualer Farbdichteverlust |
|---|---|
| 1 | 22 |
| 2 | 19 |
| 8 | 23 |
| 9 | 23 |
| 10 | 22 |
| ohne Stab. | 36 |

**Patentansprüche**

1. Farbphotographisches Aufzeichnungsmaterial, das in mindestens einer lichtempfindlichen Silberhalogenid-Emulsionsschicht, einer Zwischenschicht, einer Bildempfangsschicht und/oder einer Schutzschicht als Stabilisator mindestens eine Verbindung der Formeln, I, II oder III enthält,

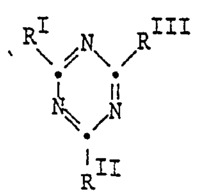 (I)

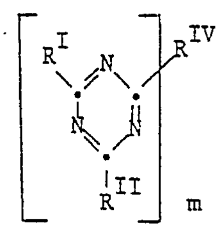 (II)

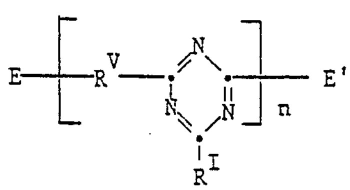 (III)

worin R$^I$, R$^{II}$ und R$^{III}$ unabhängig voneinander entweder

a) eine Gruppe der Formeln IV, V, VI oder VIa sind,

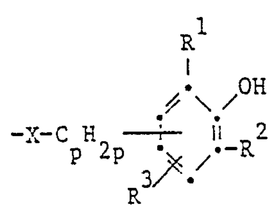 (IV)

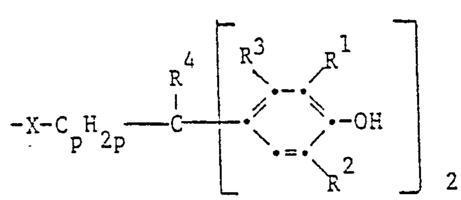 (V)

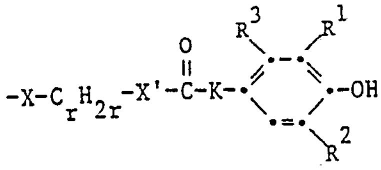

(VI)

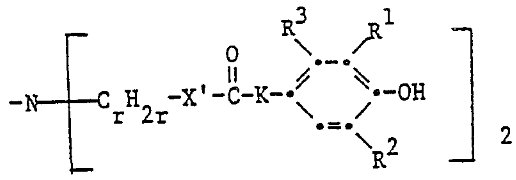

(VIa)

worin

p null oder eine Zahl von 1-12 ist (XII)

r eine ganze Zahl von 2-6 ist,

X —O—, —S— oder —$NR^5$— bedeutet,

X' —O— oder —$NR^5$— bedeutet

K eine direkte Bindung, $C_1$-$C_6$-Alkylen oder ein Gruppe der Formel

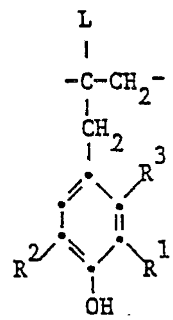

bedeutet,

$R^1$ Wasserstoff, $C_1$-$C_8$-Alkyl, $C_5$-$C_8$-Cycloalkyl, $C_7$-$C_9$-Phenylalkyl, Phenyl oder $C_7$-$C_{10}$-Alkylphenyl bedeutet,

$R^2$ $C_1$-$C_8$-Alkyl, $C_5$-$C_8$-Cycloalkyl, $C_7$-$C_9$-Phenylalkyl, Phenyl oder $C_7$-$C_{10}$-Alkylphenyl bedeutet,

$R^3$ Wasserstoff oder Methyl ist,

$R^4$ Wasserstoff, $C_1$-$C_{12}$-Alkyl, $C_5$-$C_8$-Cycloalkyl, Phenyl oder Benzyl bedeutet,

$R^5$ Wasserstoff, $C_1$-$C_{12}$-Alkyl, $C_1$-$C_{12}$-Cycloalkyl, Allyl oder Benzyl ist, und

L —ON, —$COCH_3$ oder —$SO_2CH_3$ bedeutet,

oder

b) eine Gruppe der Formeln VII bis XV sind,

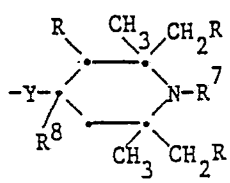

(VII)

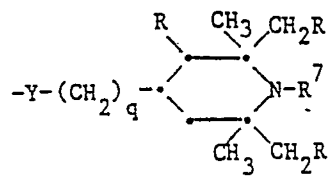

(VIII)

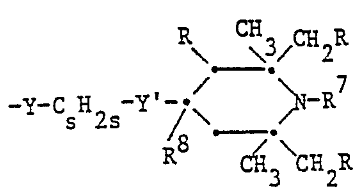

(IX)

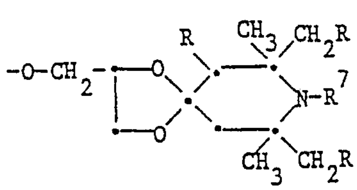

(X)

(Fortsetzung)

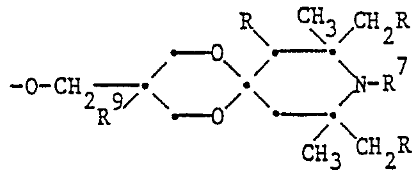
(XI)

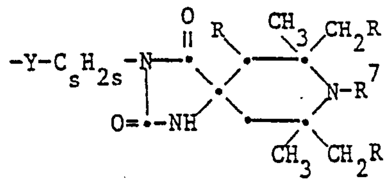

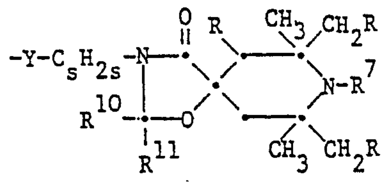
(XIII)

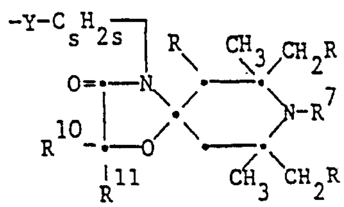
(XIV)

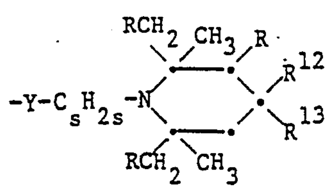
(XV)

worin

Y und Y' unabhängig voneinander —O— oder —N($R^6$)— bedeuten und

$R^6$ Wasserstoff, $C_1$-$C_{18}$-Alkyl, $C_3$-$C_6$-Alkenyl, $C_5$-$C_{12}$-Cycloalkyl, $C_7$-$C_{12}$-Phenylalkyl, $C_3$-$C_{12}$-Alkoxyalkyl, $C_4$-$C_{12}$-Dialkylaminoalkyl, eine Gruppe —$C_sH_{2s}$—X'—CO—$R^{14}$ oder

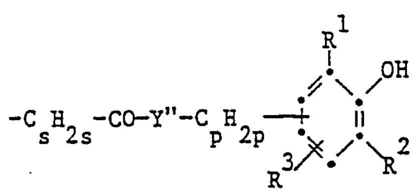

oder eine Gruppe der Formel XVI bedeutet,

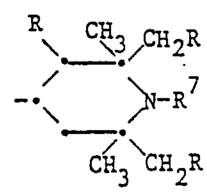
(XVI)

R Wasserstoff oder Methyl ist,

$R^7$ Hydroxyl, $C_1$-$C_{12}$-Alkyl, $C_3$-$C_6$-Alkenyl, $C_3$-$C_4$-Alkinyl, $C_7$-$C_{12}$-Phenylalkyl, Glycidyl, durch Halogen, —CN, —$COOR^{15}$ oder —$CON(R^{16})$ $(R^{17})$ substituiertes $C_1$-$C_4$-Alkyl, eine Gruppe —CO-$R^{14}$, —CO—$OR^{15}$, —CO—$N(R^{16})$ $(R^{17})$, —$CH_2$—$CH(R^{18})$—$OR^{19}$, —SO—$R^{20}$, —$SO_2$—$R^{20}$, —$OR^{15}$ oder —OOC—$R^{14}$ bedeutet,

$R^8$ Wasserstoff, —CN, —$COOR^{15}$, $CONH_2$ oder —$CON(R^{16})$ $(R^{17})$ bedeutet,

$R^9$ Methyl oder Ethyl ist,

$R^{10}$ Wasserstoff, $C_1$-$C_{12}$-Alkyl, $C_5$-$C_8$-Cycloalkyl oder Benzyl und

$R^{11}$ $C_1$-$C_{12}$-Alkyl, $C_5$-$C_8$-Cycloalkyl oder Phenyl ist, oder

$R^{10}$ und $R^{11}$ zusammen mit dem C-Atom, an das sie gebunden sind, einen $C_5$-$C_{12}$-Cycloalkan- oder Alkylcycloalkanring bilden,

$R^{12}$ Wasserstoff, —$OR^{21}$, —OOC—$R^{14}$, oder —$N(R^{22})$—CO—$R^{14}$ und

$R^{13}$ Wasserstoff, —CN, —$COOR^{15}$, —$CONH_2$ oder —$CON(R^{16})$ $(R^{17})$ bedeutet

oder

$R^{12}$ und $R^{13}$ zusammen eine Gruppe der Formeln

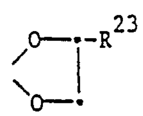

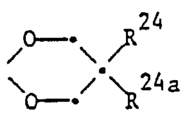

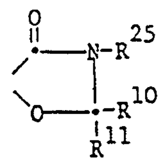

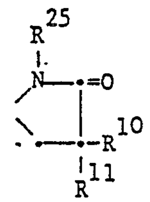

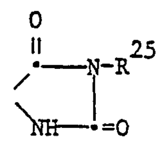

q 1 oder 2 ist,

s eine ganze Zahl von 2-6 ist,

Y″ —O— oder —$NR^{5a}$— bedeutet, worin $R^{5a}$ die Bedeutung von $R^5$ hat oder eine Gruppe der Formel XVI ist,

$R^{14}$ Wasserstoff, $C_1$-$C_{12}$-Alkyl, $C_2$-$C_6$-Alkenyl, Chlormethyl, $C_5$-$C_{12}$-Cycloalkyl, $C_7$-$C_{12}$-Phenylalkyl, Phenyl, $C_7$-$C_{10}$-Alkylphenyl oder durch 1 oder 2 $C_1$-$C_4$-Alkylgruppen und eine Hydroxygruppe substituiertes Phenyl, Phenylmethyl oder Phenylethyl bedeutet,

$R^{15}$ $C_1$-$C_{12}$-Alkyl, Allyl, Benzyl oder Cyclohexyl bedeutet,

$R^{16}$ $C_1$-$C_{12}$-Alkyl, Allyl, Cyclohexyl, Benzyl oder Phenyl, und

$R^{17}$ Wasserstoff, $C_1$-$C_8$-Alkyl oder Allyl bedeuten oder

$R^{16}$ und $R^{17}$ zusammen mit dem N-Atom einen 5- oder 6-gliedrigen heterocyclischen Ring bilden,

$R^{18}$ Wasserstoff, $C_1$-$C_4$-Alkyl, $C_2$-$C_{13}$-Alkoxymethyl, Phenyl oder Phenoxymethyl bedeutet,

$R^{19}$ Wasserstoff, $C_1$-$C_{12}$-Alkyl, —CO—$R^{14}$ oder —CO—$N(R^{16})(R^{17})$ bedeutet,

$R^{20}$ $C_1$-$C_{12}$-Alkyl, Phenyl oder $C_7$-$C_{22}$-Alkylaryl bedeutet,

$R^{21}$ Wasserstoff, $C_1$-$C_{12}$-Alkyl, Allyl oder Benzyl ist,

$R^{22}$ Wasserstoff, $C_1$-$C_{12}$-Alkyl, $C_5$-$C_{12}$-Cycloalkyl oder Benzyl ist,

$R^{23}$ Wasserstoff, Methyl oder Ethyl ist,

$R^{24}$ und $R^{24a}$ unabhängig voneinander H oder $C_1$-$C_4$-Alkyl sind und

$R^{25}$ Wasserstoff, $C_1$-$C_{12}$-Alkyl, $C_3$-$C_{12}$-Alkoxyalkyl, $C_5$-$C_{12}$-Cycloalkyl Allyl oder Benzyl bedeutet,

oder

c) eine Gruppe —$OR^{26}$, —$SR^{27}$, —$P(O)(OR^{28})_2$ oder —$N(R^{29})(R^{30})$ sind,

worin

$R^{26}$ Wasserstoff, $C_1$-$C_{18}$-Alkyl, $C_3$-$C_6$-Alkenyl, $C_3$-$C_{12}$-Alkoxyalkyl, $C_4$-$C_{12}$-Dialkylaminoalkyl, Phenyl, $C_7$-$C_{22}$-Alkylaryl, $C_7$-$C_9$-Phenylalkyl oder $C_5$-$C_{12}$-Cycloalkyl bedeutet,

$R^{27}$ $C_1$-$C_{18}$-Alkyl, $C_5$-$C_{12}$-Cycloalkyl, Phenyl, $C_7$-$C_{12}$-Alkylphenyl, $C_7$-$C_9$-Phenylalkyl, —$CH_2COOR^{31}$ oder —$CH_2CH_2COOR^{31}$ und $R^{31}$ Wasserstoff oder $C_1$-$C_{12}$-Alkyl bedeutet,

$R^{28}$ $C_1$-$C_{12}$-Alkyl, Phenyl, Tolyl, Benzyl oder eine Gruppe der Formel XVI bedeutet,

$R^{29}$ und $R^{30}$ unabhängig voneinander Wasserstoff, $C_1$-$C_{12}$-Alkyl, durch $C_1$-$C_4$-Alkoxy, $R^{14}$—COO— oder $C_2$-$C_8$-Dialkylamino substituiertes $C_2$-$C_4$-Alkyl, $C_5$-$C_{12}$-Cycloalkyl, $C_3$-$C_6$-Alkenyl oder $C_7$-$C_9$-Phenylalkyl bedeuten oder

$R^{29}$ und $R^{30}$ zusammen mit dem N-Atom, an das sie gebunden sind, einen 5- oder 6-gliedrigen heterocyclischen Ring bilden

und

$R^{III}$ auch eine Gruppe der Formel XVII

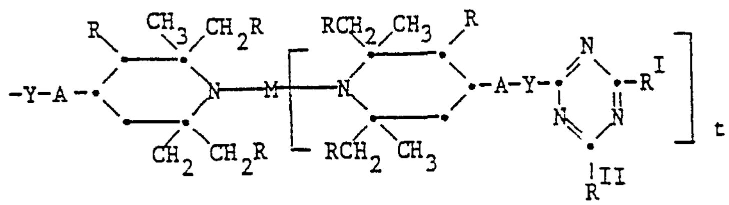

(XVII)

sein kann, worin t 1, 2 oder 3 ist,

A eine direkte Bindung, eine Gruppe $—CH_2)_q—$ oder eine Gruppe $—Y'—C_sH_{2s}—$ ist und

M, wenn t = 1 ist, eine zweiwertige Gruppe ist und $C_2$-$C_{12}$-Alkylen, $C_4$-$C_8$-Alkenylen, Xylylen oder einen Rest der Formel

$—CH_2—C≡C—CH_2—$ 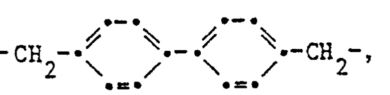, 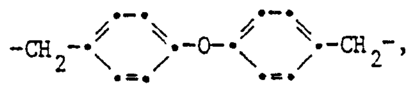,

$-CH_2-COO-B-OOC-CH_2-$, $-CH_2-CH(OH)-CH_2-$, $-CH_2CH(OH)CH_2-D-CH_2CH(OH)CH_2-$, $-CH_2-CH(R^{18})-OOC-R^{33}-COO-CH(R^{18})-CH_2-$ oder

$-CO-NH-G-NH-CO-$

darstellt, worin B $C_2$-$C_{12}$-Alkylen, $C_4$-$C_8$-Oxaalkylen oder Cyclohexylen ist, D einen zweiwertigen Rest der Formel $—O—R^{32}—O—$ oder $—OOC—R^{33}—COO—$ darstellt, worin $R^{32}$ $C_2$-$C_{12}$-Alkylen, $C_6$-$C_{12}$-Cycloalkylen, $C_6$-$C_{12}$-Arylen oder -Phenylen-Z-Phenylen- und Z —O—, $—CH_2—$,

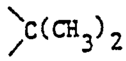

oder $—SO_2$ bedeutet, $R^{33}$ eine direkte Bindung, $C_1$-$C_{12}$-Alkylen, $C_2$-$C_6$-Alkenylen, $C_6$-$C_{12}$-Cycloalkylen oder Cycloalkenylen oder $C_6$-$C_{12}$-Arylen bedeutet und G einen zweiwertigen aliphatischen, cycloaliphatischen, aromatischen oder aromatisch-aliphatischen Rest mit 6-15 C-Atomen bedeuten,

M, wenn t = 2 ist, einen dreiwertigen Rest der Formel

$T—[CH_2CH(OH)CH_2—]—_3$ oder $R^{34}—[COO—CH(R^{18})—CH_2—]—_3$

darstellt, worin T ein dreiwertiger Rest der Formeln

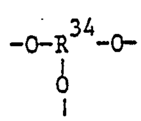 oder 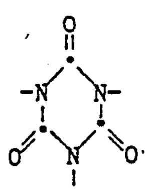 ist

und $R^{34}$ ein dreiwertiger aliphatischer Kohlenwasserstoffrest mit 3-10 Kohlenstoffatomen oder ein dreiwertiger aromatischer Kohlenwasserstoffrest mit 6-10 C-Atomen ist, und

M, wenn t = 3 ist, einen vierwertigen Rest der Formel

$Q—[CH_2CH(OH)CH_2—]—4$ oder $R^{35}—[—COO—CH(R^{18})—CH_2—]—4$

darstellt, worin

Q eine Gruppe der Formel

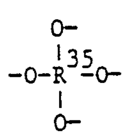 oder 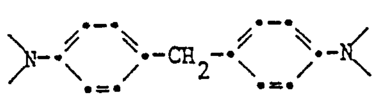

darstellt, und $R^{35}$ einen vierwertigen aliphatischen Kohlenwasserstoffrest mit 4-10 C-Atomen oder einen vierwertigen aromatischen Kohlenwasserstoffrest mit 6-12 C-Atomen bedeutet,

$R^{IV}$ der m-wertige Rest eines Polyamines oder eines Polyols ist, und

m eine ganze Zahl von 2-6 ist,
$R^V$ eine zweiwertige Gruppe —Y—$R^{36}$—Y— oder

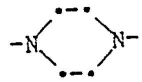 ist,

worin $R^{36}$ $C_2$-$C_{12}$-Alkylen, $C_5$-$C_{12}$-Cycloalkylen, $C_8$-$C_{12}$-Aralkylen, $C_6$-$C_{12}$-Arylen, durch O oder $NR^5$ unterbrochenes $C_4$-$C_8$-Alkylen oder eine Gruppe der Formel

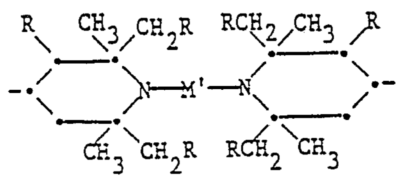 oder

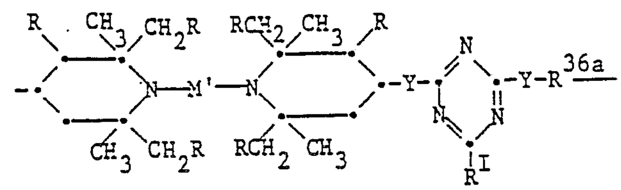

darstellt, worin

M' eine zweiwertige Gruppe der Formel M ist und

$R^{36a}$ $C_2$-$C_{12}$-Alkylen, $C_5$-$C_{12}$-Cycloalkylen, $C_8$-$C_{12}$-Aralkylen, $C_6$-$C_{12}$-Arylen oder durch O oder $NR^5$ unterbrochenes $C_4$-$C_8$-Alkylen bedeutet,

n 2 bis 50 ist und E und E' Endgruppen darstellen,

wobei mindestens einer der Reste $R^I$, $R^{II}$, $R^{III}$ eine sterisch gehinderte Phenolgruppe enthält und mindestens einer der Reste $R^I$, $R^{II}$, $R^{III}$, $R^{IV}$, $R^V$ einen Polyalkylpiperidinrest enthält.

2. Farbphotographisches Aufzeichnungsmaterial gemäss Anspruch 1, enthaltend als Stabilisator eine Verbindung der Formel I, II oder III, welche in ihrem Molekül mindestens eine sterisch gehinderte Phenolgruppe und mindestens eine N-substituierte 2,2,6,6-Tetramethylpiperidingruppe enthält.

3. Farbphotographisches Aufzeichnungsmaterial gemäss Anspruch 1, enthaltend als Stabilisator eine Verbindung der Formel I, worin R', R'' und R''' unabhängig voneinander

a) eine Gruppe der Formel IVa sind,

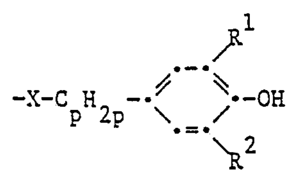 (IVa)

worin p eine ganze Zahl von 1 bis 4 ist und X, $R^1$ und $R^2$ die in Anspruch 1 gegebene Bedeutung haben,

b) eine Gruppe der Formeln VIIa oder XVa sind

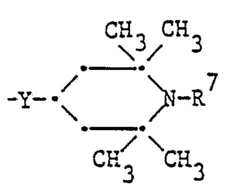 (VIIa) 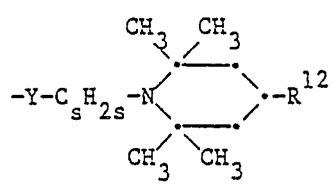 (XVa)

worin Y —O— oder —$NR^6$— bedeutet und $R^6$, $R^7$, $R^{12}$ und s die in Anspruch 1 gegebene Bedeutung haben, oder

c) eine Gruppe —$OR^{26}$, —$SR^{27}$, —P(O) $(OR^{28})_2$ oder —$N(R^{29})(R^{30})$ sind, worin $R^{26}$, $R^{27}$, $R^{28}$, $R^{29}$ und $R^{30}$ die in Anspruch 1 gegebene Bedeutung haben,

wobei mindestens einer der Reste $R^I$, $R^{II}$, $R^{III}$ eine sterisch gehinderte Phenolgruppe enthält und mindestens einer der Reste $R^I$, $R^{II}$, $R^{III}$ eine N-substituierte 2,2,6,6-Tetramethylpiperidingruppe enthält.

4. Farbphotographisches Aufzeichnungsmaterial gemäss Anspruch 3, enthaltend als Stabilisator eine Verbindung der Formel I, worin $R^I$, $R^{II}$ und $R^{III}$ unabhängig voneinander entweder

a) eine Gruppe der Formel IVa sind, worin $R^1$ Wasserstoff oder $C_1$-$C_4$-Alkyl und $R^2$ $C_1$-$C_4$-Alkyl sind und X und p die in Anspruch 3 gegebene Bedeutung haben, oder

b) eine Gruppe der Formel VIIa oder XVa sind, worin Y —O— oder —$NR^6$— bedeutet und $R^6$ Wasserstoff, $C_1$-$C_8$-Alkyl, Allyl, Cyclohexyl, Benzyl oder eine Gruppe —$C_sH_{2s}$—X'—CO—$R^{14}$ ist, worin s = 2 oder 3 ist, X' —O— ist und $R^{14}$ durch 1 oder 2 $C_1$-$C_4$-Alkylgruppen und eine Hydroxylgruppe substituiertes Phenyl, Phenylmethyl oder Phenylethyl bedeutet, $R^7$ Methyl, Allyl, Benzyl, 2-Hydroxyethyl, Acetyl, Acryloyl oder eine Gruppe —$CON(R^{16})(R^{17})$ ist, worin $R^{16}$ $C_1$-$C_8$-Alkyl, Cyclohexyl oder Phenyl und $R^{17}$ Wasserstoff oder $C_1$-$C_8$-Alkyl bedeuten, und $R^{12}$ Wasserstoff ist, oder

c) eine Gruppe —$OR^{26}$, —$SR^{27}$ oder —$N(R^{29})(R^{30})$ sind, worin $R^{26}$ $C_1$-$C_{12}$-Alkyl, Phenyl, $C_7$-$C_{22}$-Alkylaryl, Benzyl oder Cyclohexyl bedeutet, $R^{27}$ $C_1$-$C_{12}$-Alkyl, Cyclohexyl, Phenyl, —$CH_2COO(C_1$-$C_{12}$-Alkyl) oder —$CH_2CH_2COO(C_1$-$C_{12}$-Alkyl) bedeutet und $R^{29}$ und $R^{30}$ Wasserstoff, $C_1$-$C_{12}$ Alkyl, Benzyl oder Allyl bedeuten.

5. Farbphotographisches Aufzeichnungsmaterial gemäss Anspruch 1, enthaltend als Stabilisator eine Verbindung der Formel II, worin m = 2 ist und $R^{IV}$ eine zweiwertige Gruppe der Formel

$-N(R^6)-R^{37}-N(R^6)-$ oder 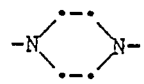 ist,

worin $R^{37}$ $C_2$-$C_{12}$-Alkylen, durch —O— oder —$NR^5$— unterbrochenes $C_4$-$C_8$-Alkylen, $C_6$-$C_{15}$-Arylen, $C_8$-$C_{12}$-Aralkylen oder $C_5$-$C_{12}$-Cycloalkylen ist und $R^5$ und $R^6$ die in Anspruch 1 gegebene Bedeutung haben.

6. Farbphotographisches Aufzeichnungsmaterial gemäss Anspruch 1, enthaltend als Stabilisator eine Verbindung der Formel II, worin m = 3 und $R^{IV}$ eine dreiwertige Gruppe der Formel

$$-N(R^6)-(CH_2)_a-\overset{|}{N}(R^6)-(CH_2)_a-N(R^6)- \quad \text{ist,}$$

oder m = 4 und $R^{IV}$ eine vierwertige Gruppe der Formel

$$-N(R^6)-(CH_2)_a-\overset{|}{N}(R^6)-(CH_2)_b-\overset{|}{N}(R^6)-(CH_2)_a-N(R^6)-$$

ist, worin a 2 oder 3 und b 2-12 bedeutet und $R^6$ die in Anspruch 1 gegebene Bedeutung hat.

7. Farbphotographisches Aufzeichnungsmaterial gemäss Anspruch 1, enthaltend als Stabilisator eine Verbindung der Formel III, worin $R^V$ eine zweiwertige Gruppe —$N(R^6)$—$R^{36a}$—$N(R^6)$— ist, in welcher $R^{36a}$ und $R^6$ die in Anspruch 1 gegebene Bedeutung haben.

8. Farbphotographisches Aufzeichnungsmaterial gemäss Anspruch 7, dadurch gekennzeichnet, dass in der Verbindung der Formel III die Endgruppe E vorwiegend Wasserstoff ist und die Endgruppe E' vorwiegend eine Gruppe —$N(R^6)$—$R^{36a}$—$NHR^6$ ist.

9. Farbphotographisches Aufzeichnungsmaterial gemäss Anspruch 7, enthaltend als Stabilisator eine Verbindung der Formel III, worin

$R^I$ eine Gruppe

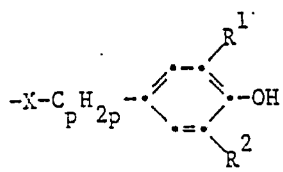

und

$R^V$ eine Gruppe

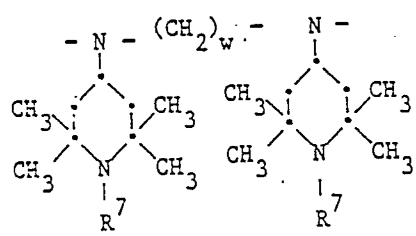

bedeutet, worin w 2-6 ist und $R^1$, $R^2$, X, p und $R^7$ die in Anspruch 1 gegebene Bedeutung haben.

10. Farbphotographisches Aufzeichnungsmaterial gemäss Anspruch 9, enthaltend als Stabilisator eine Verbindung der Formel III, worin

$R^I$ eine Gruppe

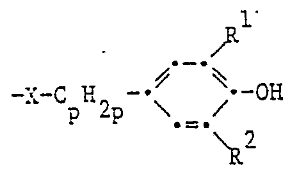

und

$R^V$ eine Gruppe

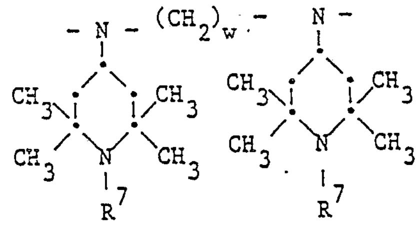

bedeutet, worin p 1-4 ist, X —O—, —S—, —NH— oder —N($C_1$-$C_4$-Alkyl)— bedeutet, $R^1$ Wasserstoff oder $C_1$-$C_4$-Alkyl und $R^2$ $C_1$-$C_4$-Alkyl ist, w 2-6 ist und $R^7$ Methyl, Allyl, Benzyl, 2-Hydroxyethyl, Acetyl, Aryloyl oder eine Gruppe —CON($R^{16}$) ($R^{17}$) ist, worin $R^{16}$ $C_1$-$C_8$-Alkyl, Cyclohexyl oder Phenyl und $R^{17}$ Wasserstoff oder $C_1$-$C_8$-Alkyl bedeutet.

11. Farbphotographisches Aufzeichnungsmaterial gemäss Anspruch 1, dadurch gekennzeichnet, dass es neben einem Stabilisator der Formel I, II oder III ein Lichtschutzmittel aus der Klasse der Ultraviolett-Absorber enthält.

12. Farbphotographisches Aufzeichnungsmaterial gemäss Anspruch 1, gekennzeichnet durch einen Gehalt an 1 bis 2 000, vorzugsweise 100 bis 800 mg pro $m^2$ an Verbindung der Formel I, II oder III.

13. Verwendung von s-Triazinverbindungen der Formel I, II oder III, wie in Anspruch 1 definiert, als Stabilisatoren für farbphotographische Aufzeichnungsmaterialien.

**Claims**

1. A colour-photographic recording material which, in at least one light-sensitive silver halide emulsion layer, one intermediate layer, one image-receiving layer and/or one protective layer, contains, as a stabilizer, at least one compound of the formulae I, II or III

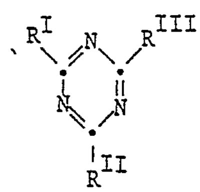

(I)

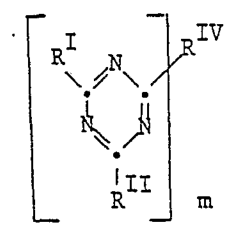

(II)

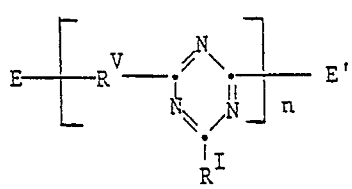

(III)

in which $R^1$, $R^{II}$ and $R^{III}$, independently of each other, are either

a) a group of the formulae IV, V, VI or VIa

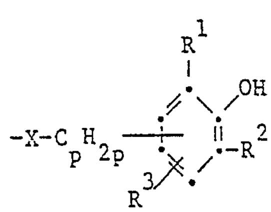

(IV)

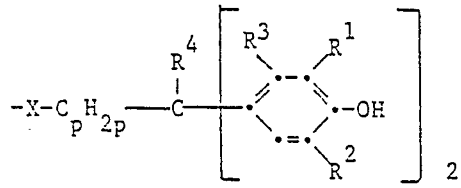

(V)

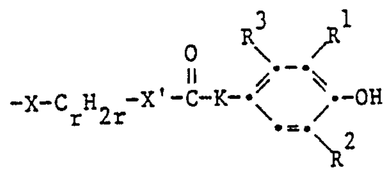

(VI)

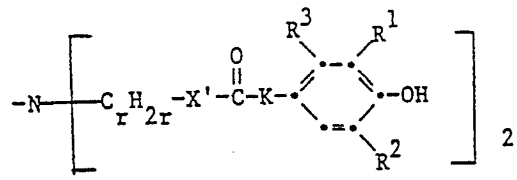

(VIa)

in which

p is zero or a number from 1 to 12,
r is an integer from 2 to 6,
X is —O—, —S— or $—NR^5—$,
X' is —O— or $—NR^5—$,
K is a direct bond, $C_1$-$C_6$-alkylene or a group of the formula

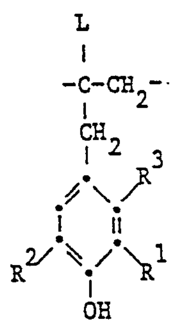

,

$R^1$ is hydrogen, $C_1$-$C_8$-alkyl, $C_5$-$C_8$-cycloalkyl, $C_7$-$C_9$-phenylalkyl, phenyl or $C_7$-$C_{10}$-alkylphenyl,
$R^2$ is $C_1$-$C_8$-alkyl, $C_5$-$C_8$-cycloalkyl, $C_7$-$C_9$-phenylalkyl, phenyl or $C_7$-$C_{10}$-alkylphenyl,
$R^3$ is hydrogen or methyl,
$R^4$ is hydrogen, $C_1$-$C_{12}$-alkyl, $C_5$-$C_8$-cycloalkyl, phenyl or benzyl,
$R^5$ is hydrogen, $C_1$-$C_{12}$-alkyl, $C_1$-$C_{12}$-cycloalkyl, allyl or benzyl, and
L is —CN, $—COCH_3$ or $—SO_2CH_3$,

or

b) a group of the formulae VII to XV

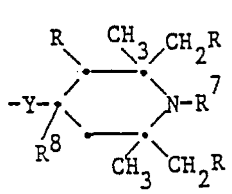

(VII)

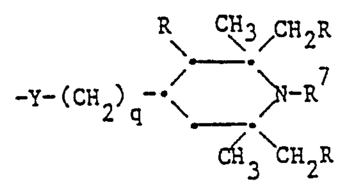

(VIII)

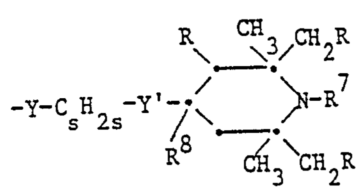

(IX)

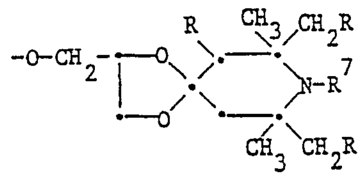

(X)

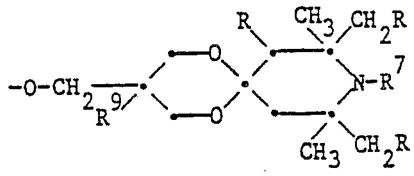

(XI)

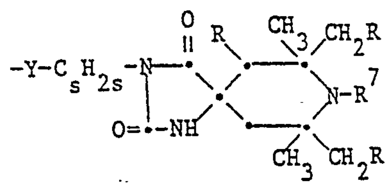

(XII)

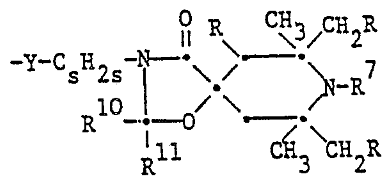

(XIII)

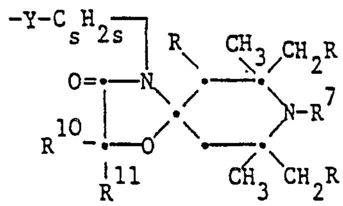

(XIV)

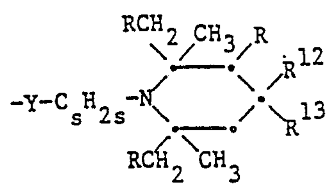

(XV)

in which

Y and Y', independently of each other, are —O— or —N($R^6$)—,

$R^6$ is hydrogen, $C_1$-$C_{18}$—alkyl, $C_3$-$C_6$-alkenyl, $C_5$-$C_{12}$-cycloalkyl, $C_7$-$C_{12}$-phenylalkyl, $C_3$-$C_{12}$-alkoxyalkyl, $C_4$-$C_{12}$-dialkylaminoalkyl, a group —$C_sH_{2s}$—X'—CO—$R^{14}$ or

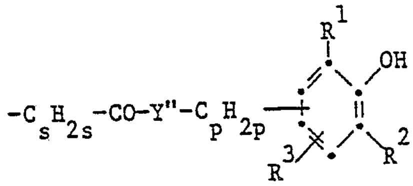

or a group of the formula XVI

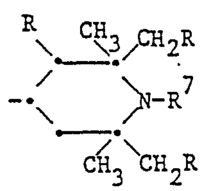

(XVI)

R is hydrogen or methyl,

$R^7$ is hydroxyl, $C_1$-$C_{12}$-alkyl, $C_3$-$C_6$-alkenyl, $C_3$-$C_4$-alkynyl, $C_7$-$C_{12}$-phenylalkyl, glycidyl, $C_1$-$C_4$-alkyl which is substituted by halogen, —CN, —$COOR^{15}$ or —$CON(R^{16})$ $(R^{17})$, a group —CO—$R^{14}$, —CO—$OR^{15}$, —CO—$N(R^{16})$ $(R^{17})$, —$CH_2$—$CH(R^{18})$—$OR^{19}$, —SO—$R^{20}$, —$SO_2$—$R^{20}$, —$OR^{15}$ or —OOC—$R^{14}$,

$R^8$ is hydrogen, —CN, —$COOR^{15}$, $CONH_2$ or —$CON(R^{16})$ $(R^{17})$,

$R^9$ is methyl or ethyl,

$R^{10}$ is hydrogen, $C_1$-$C_{12}$-alkyl, $C_5$-$C_8$-cycloalkyl or benzyl, and

$R^{11}$ is $C_1$-$C_{12}$-alkyl, $C_5$-$C_8$-cycloalkyl or phenyl, or

$R^{10}$ and $R^{11}$, together with the C atom to which they are bonded, form a $C_5$-$C_{12}$-cycloalkane or alkylcycloalkane ring,

$R^{12}$ is hydrogen, —$OR^{21}$, —OOC—$R^{14}$ or —$N(R^{22})$—CO—$R^{14}$, and

$R^{13}$ is hydrogen, —CN, —$COOR^{15}$, —$CONH_2$ or —$CON(R^{16})$ $(R^{17})$,

or

$R^{12}$ and $R^{13}$ are together a group of the formulae

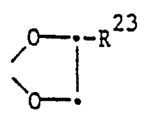

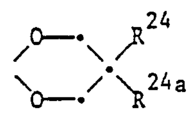

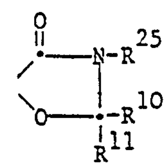

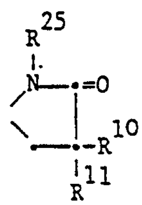

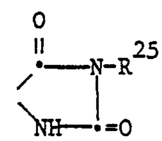

q is 1 or 2,

s is an integer from 2 to 6,

Y″ is —O— or —$NR^{5a}$—, in which $R^{5a}$ is defined in the same way as $R^5$ or is a group of the formula XVI,

$R^{14}$ is hydrogen, $C_1$-$C_{12}$-alkyl, $C_2$-$C_6$-alkenyl, chloromethyl, $C_5$-$C_{12}$-cycloalkyl, $C_7$-$C_{12}$-phenylalkyl, phenyl, $C_7$-$C_{10}$-alkylphenyl or phenyl, phenylmethyl or phenylethyl which is substituted by 1 or 2 $C_1$-$C_4$-alkyl groups and a hydroxyl group,

$R^{15}$ is $C_1$-$C_{12}$-alkyl, allyl, benzyl or cyclohexyl,

$R^{16}$ is $C_1$-$C_{12}$-alkyl, allyl, cyclohexyl, benzyl or phenyl, and

$R^{17}$ is hydrogen, $C_1$-$C_8$-alkyl or allyl, or

$R^{16}$ and $R^{17}$, together with the N atom, are a 5- or 6-membered heterocyclic ring,

$R^{18}$ is hydrogen, $C_1$-$C_4$-alkyl, $C_2$-$C_{13}$-alkoxymethyl, phenyl or phenoxymethyl,

$R^{19}$ is hydrogen, $C_1$-$C_{12}$-alkyl, —CO—$R^{14}$ or —CO—$N(R^{16})$ $(R^{17})$,

$R^{20}$ is $C_1$-$C_{12}$-alkyl, phenyl or $C_7$-$C_{22}$-alkylaryl,

$R^{21}$ is hydrogen, $C_1$-$C_{12}$-alkyl, allyl or benzyl,

$R^{22}$ is hydrogen, $C_1$-$C_{12}$-alkyl, $C_5$-$C_{12}$-cycloalkyl or benzyl,

$R^{23}$ is hydrogen, methyl or ethyl,

$R^{24}$ and $R^{24a}$, independently of each other, are H or $C_1$-$C_4$-alkyl, and

$R^{25}$ is hydrogen, $C_1$-$C_{12}$-alkyl, $C_3$-$C_{12}$-alkoxyalkyl, $C_5$-$C_{12}$-cycloalkyl, allyl or benzyl,

or

c) a group —$OR^{26}$, —$SR^{27}$, —$P(O)$ $(OR^{28})_2$ or —$N(R^{29})$ $(R^{30})$,

in which

$R^{26}$ is hydrogen, $C_1$-$C_{18}$-alkyl, $C_3$-$C_6$-alkenyl, $C_3$-$C_{12}$-alkoxyalkyl, $C_4$-$C_{12}$-dialkylamino-alkyl, phenyl, $C_7$-$C_{22}$-alkylaryl, $C_7$-$C_9$-phenylalkyl or $C_5$-$C_{12}$-cycloalkyl,

$R^{27}$ is $C_1$-$C_{18}$-alkyl, $C_5$-$C_{12}$-cycloalkyl, phenyl, $C_7$-$C_{12}$-alkylphenyl, $C_7$-$C_9$-phenylalkyl, —$CH_2COOR^{31}$ or —$CH_2CH_2COOR^{31}$,

$R^{31}$ is hydrogen or $C_1$-$C_{12}$-alkyl,

$R^{28}$ is $C_1$-$C_{12}$-alkyl, phenyl, tolyl, benzyl or a group of the formula XVI,

$R^{29}$ and $R^{30}$, independently of each other, are hydrogen, $C_1$-$C_{12}$-alkyl, $C_2$-$C_4$-alkyl which is substituted by $C_1$-$C_4$-alkoxy, $R^{14}$—COO— or $C_2$-$C_8$-dialkylamino, $C_5$-$C_{12}$-cycloalkyl, $C_3$-$C_6$-alkenyl or $C_7$-$C_9$-phenylalkyl, or

$R^{29}$ and $R^{30}$, together with the N atom to which they are bonded, form a 5- or 6-membered heterocyclic ring,

and

$R^{III}$ can also be a group of the formula XVII

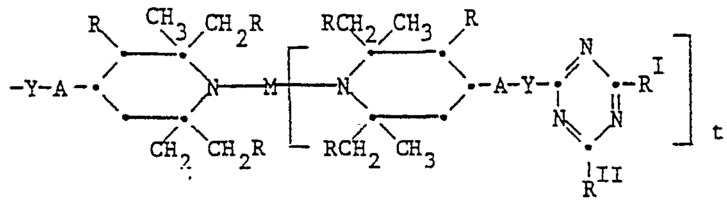

(XVII)

in which t is 1, 2 or 3,

A is a direct bond, a $-(CH_2)_q$-group or a $-Y'-C_sH_{2s}$-group, and

M, if t = 1, is a divalent group, $C_2$-$C_{12}$-alkylene, $C_4$-$C_8$-alkenylene, xylylene or a radical of the formula

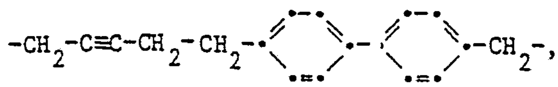 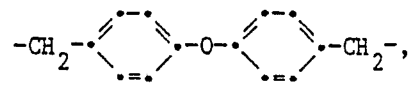

$-CH_2-COO-B-OOC-CH_2-$, $-CH_2-CH(OH)-CH_2-$, $-CH_2CH(OH)CH_2-D-CH_2CH(OH)CH_2-$, $-CH_2-CH(R^{18})-OOC-R^{33}-COO-CH(R^{18})-CH_2-$ or $-CO-NH-G-NH-CO-$

in which B is $C_2$-$C_{12}$-alkylene, $C_4$-$C_8$-oxaalkylene or cyclohexylene, D is a divalent radical of the formula $-O-R^{32}-O-$ or $-OOC-R^{33}-COO-$, in which $R^{32}$ is $C_2$-$C_{12}$-alkylene, $C_6$-$C_{12}$-cycloalkylene, $C_6$-$C_{12}$-arylene or -phenylene-Z-phenylene, and Z is $-O-$, $-CH_2-$,

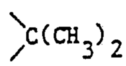

or $-SO_2$, $R^{33}$ is a direct bond, $C_1$-$C_{12}$-alkylene, $C_2$-$C_6$-alkenylene, $C_6$-$C_{12}$-cycloalkylene or cycloalkenylene or $C_6$-$C_{12}$-arylene, and G is a divalent aliphatic, cycloaliphatic, aromatic or aromatic-aliphatic radical having 6-15 C atoms,

M, if t = 2, is a trivalent radical of the formula

$$T-[-CH_2CH(OH)\ CH_2-]-_3 \quad \text{or} \quad R^{34}-[-COO-CH(R^{18})-CH_2-]-_3$$

in which T is a trivalent radical of the formulae

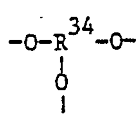 or 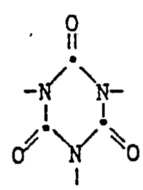

and $R^{34}$ is a trivalent aliphatic hydrocarbon radical having 3-10 carbon atoms or a trivalent aromatic hydrocarbon radical having 6-10 C atoms, and

M, if t = 3, is a tetravalent radical of the formula

$$Q-[-CH_2CH(OH)CH_2-]-_4 \quad \text{or} \quad R^{35}-[-COO-CH(R^{18})-CH_2-]-_4$$

in which

Q is a group of the formula

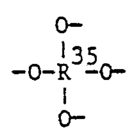 or 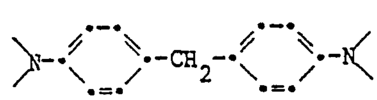

and $R^{35}$ is a tetravalent aliphatic hydrocarbon radical having 4-10 C atoms or a tetravalent aromatic hydrocarbon radical having 6-12 C atoms,

$R^{IV}$ is the m-valent radical of a polyamine or of a polyol, and

m is an integer from 2 to 6,

$R^V$ is a divalent group $-Y-R^{36}-Y-$ or

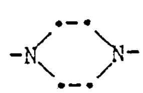

in which $R^{36}$ is $C_2$-$C_{12}$-alkylene, $C_5$-$C_{12}$-cycloalkylene, $C_8$-$C_{12}$-aralkylene, $C_6$-$C_{12}$-arylene, $C_4$-$C_8$-alkylene which is interrupted by O or $NR^5$ or a group of the formula

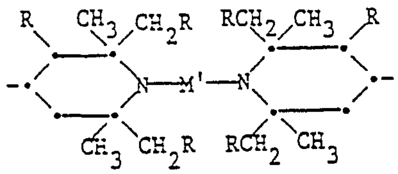

or

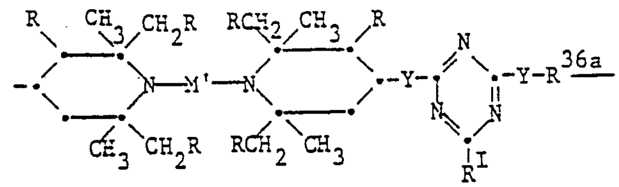

in which

M' is a divalent group of the formula M, and

$R^{36a}$ is $C_2$-$C_{12}$-alkylene, $C_5$-$C_{12}$-cycloalkylene, $C_8$-$C_{12}$-aralkylene, $C_6$-$C_{12}$-arylene or $C_4$-$C_8$-alkylene which is interrupted by O or $NR^5$,

n is 2 to 50, and E and E' are terminal groups,

and at least one of the radicals $R^I$, $R^{II}$ and $R^{III}$ contains a sterically hindered phenol group and at least one of the radicals $R^I$, $R^{II}$, $R^{III}$, $R^{IV}$ and $R^V$ contains a polyalkylpiperidine radical.

2. A colour-photographic recording material according to claim 1, which contains, as a stabilizer, a compound which is of the formula I, II or III and which contains in its molecule at least one sterically hindered phenol group and at least one N-substituted 2,2,6,6-tetramethylpiperidine group.

3. A colour-photographic recording material according to claim 1, which contains, as a stabilizer, a compound of the formula I in which R', R'' and R''', independently of each other, are

a) a group of the formula IVa

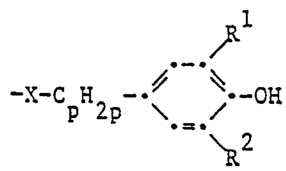

(IVa)

in which p is an integer from 1 to 4, and X, $R^1$ and $R^2$ are as defined in claim 1,

b) a group of the formulae VIIa or XVa

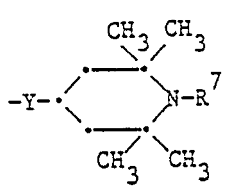

(VIIa)

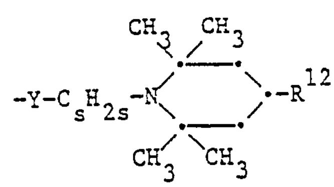

(XVa)

in which Y is —O— or —$NR^6$—, and $R^6$, $R^7$, $R^{12}$ and s are as defined in claim 1, or

c) a group —$OR^{26}$, —$SR^{27}$, —P(O) $(OR^{28})_2$ or —$N(R^{29})$ $(R^{30})$, in which $R^{26}$, $R^{27}$, $R^{28}$, $R^{29}$ and $R^{30}$ are as defined in claim 1,

and at least one of the radicals $R^I$, $R^{II}$ and $R^{III}$ contains a sterically hindered phenol group, and at least one of the radicals $R^I$, $R^{II}$ and $R^{III}$ contains an N-substituted 2,2,6,6-tetramethylpiperidine group.

4. A colour-photographic recording material according to claim 3, which contains, as a stabilizer, a compound of the formula I in which $R^I$, $R^{II}$ and $R^{III}$ , independently of each other, are either

a) a group of the formula IVa in which $R^1$ is hydrogen or $C_1$-$C_4$-alkyl, $R^2$ is $C_1$-$C_4$-alkyl, and X and p are as defined in claim 3, or

b) a group of the formula VIIa or XVa in which Y is —O— or —$NR^6$—, $R^6$ is hydrogen, $C_1$-$C_8$-alkyl, allyl, cyclohexyl, benzyl or a group —$C_sH_{2s}$—X'—CO—$R^{14}$ in which s = 2 or 3, X' is —O— and $R^{14}$ is

phenyl, phenylmethyl or phenylethyl which is substituted by 1 or 2 $C_1$-$C_4$-alkyl groups and a hydroxyl group, $R^7$ is methyl, allyl, benzyl, 2-hydroxyethyl, acetyl, acryloyl or a group $-CON(R^{16})(R^{17})$ in which $R^{16}$ is $C_1$-$C_8$-alkyl, cyclohexyl or phenyl, and $R^{17}$ is hydrogen or $C_1$-$C_8$-alkyl, and $R^{12}$ is hydrogen, or

c) a group of the formula $-OR^{26}$, $-SR^{27}$ or $-N(R^{29})(R^{30})$ in which $R^{26}$ is $C_1$-$C_{12}$-alkyl, phenyl, $C_7$-$C_{22}$-alkylaryl, benzyl or cyclohexyl, $R^{27}$ is $C_1$-$C_{12}$-alkyl, cyclohexyl, phenyl, $-CH_2COO(C_1$-$C_{12}$-alkyl) or $-CH_2CH_2COO(C_1$-$C_{12}$-alkyl), and $R^{29}$ and $R^{30}$ are hydrogen, $C_1$-$C_{12}$-alkyl, benzyl or allyl.

5. A colour-photographic recording material according to claim 1, which contains, as a stabilizer, a compound of the formula II in which m = 2 and $R^{IV}$ is a divalent group of the formula

$-N(R^6)-R^{37}-N(R^6)-$ or 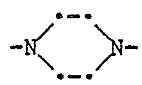

in which $R^{37}$ is $C_2$-$C_{12}$-alkylene, $C_4$-$C_8$-alkylene which is interrupted by $-O-$ or $-NR^5-$, $C_6$-$C_{15}$-arylene, $C_8$-$C_{12}$-aralkylene or $C_5$-$C_{12}$-cycloalkylene, and $R^5$ and $R^6$ are as defined in claim 1.

6. A colour-photographic recording material according to claim 1, which contains, as a stabilizer, a compound of the formula II in which m = 3 and $R^{IV}$ is a trivalent group of the formula

$$-N(R^6)-(CH_2)_a-\overset{|}{N}(R^6)-(CH_2)_a-N(R^6)-$$

or m = 4 and $R^{IV}$ is a tetravalent group of the formula

$$-N(R^6)-(CH_2)_a-\overset{|}{N}(R^6)-(CH_2)_b-\overset{|}{N}(R^6)-(CH_2)_a-N(R^6)-$$

in which a is 2 or 3, b is 2-12, and $R^6$ is as defined in claim 1.

7. A colour-photographic recording material according to claim 1, which contains, as a stabilizer, a compound of the formula III in which $R^V$ is a divalent group $-N(R^6)-R^{36a}-N(R^6)-$ in which $R^{36a}$ and $R^6$ are as defined in claim 1.

8. A colour-photographic recording material according to claim 7, wherein, in the compound of the formula III, the terminal group E is predominantly hydrogen and the terminal group E' is predominantly a group $-N(R^6)-R^{36a}-NHR^6$.

9. A colour-photographic recording material according to claim 7, which contains, as a stabilizer, a compound of the formula III in which

$R^I$ is a group

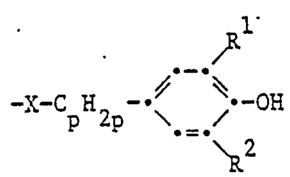

and $R^V$ is a group

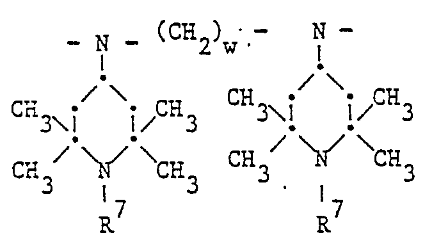

in which w is 2-6, and $R^1$, $R^2$, X, p and $R^7$ are as defined in claim 1.

10. A colour-photographic recording material according to claim 9, which contains, as a stabilizer, a compound of the formula III in which

$R^I$ is a group

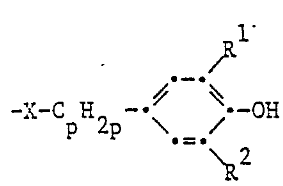

and $R^V$ is a group

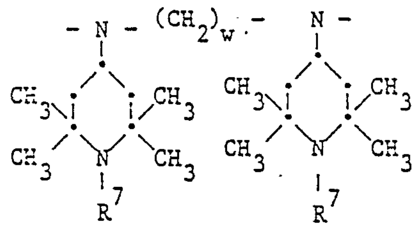

in which p is 1-4, X is —O—, —S—, —NH— or —N($C_1$-$C_4$-alkyl)—, $R^1$ is hydrogen or $C_1$-$C_4$-alkyl, $R^2$ is $C_1$-$C_4$-alkyl, w is 2-6 and $R^7$ is methyl, allyl, benzyl, 2-hydroxyethyl, acetyl, aryloyl or a group —CON($R^{16}$)($R^{17}$) in which $R^{16}$ is $C_1$-$C_8$-alkyl, cyclohexyl or phenyl and $R^{17}$ is hydrogen or $C_1$-$C_8$-alkyl.

11. A colour-photographic recording material according to claim 1, which, in addition to a stabilizer of the formula I, II or III, contains a light stabilizer from the class of the ultraviolet absorbers.

12. A colour-photographic recording material according to claim 1, which contains 1 to 2,000, preferably 100 to 800 mg of compound of the formula I, II or III per $m^2$.

13. Use of s-triazine compounds of the formula I, II or III as defined in claim 1, as stabilizers for colour-photographic recording materials.

**Revendications**

1. Matière pour enregistrement photographique en couleurs qui, dans au moins une couche d'émulsion d'halogénure d'argent photosensible, une couche intermédiaire, une couche réceptrice d'images et/ou une couche protectrice, contient, comme stabilisant, au moins un composé répondant à l'une des formules I, II et III :

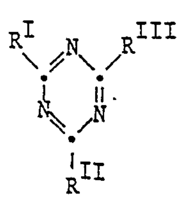

(I)

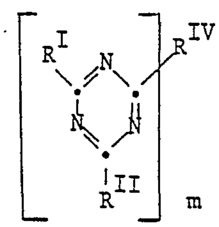

(II)

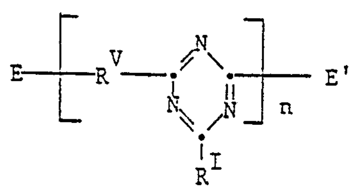

(III)

dans lesquelles $R^I$, $R^{II}$ et $R^{III}$ représentent chacun, indépendamment les uns des autres, soit :

a) un radical répondant à l'une des formules IV, V, VI et VIa :

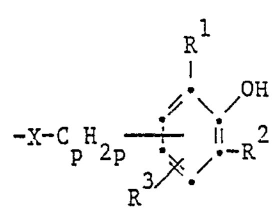

(IV)

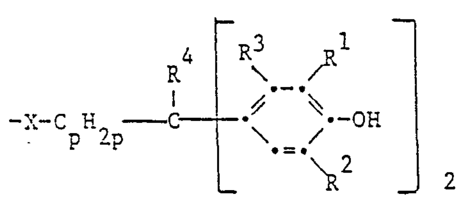

(V)

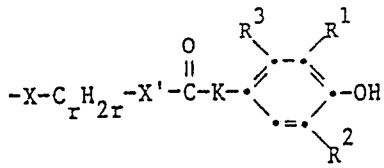

(VI)

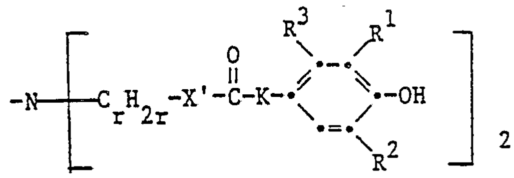

(VIa)

dans lesquelles :

p représente un nombre de 0 à 12,

r représente un nombre entier de 2 à 6,

X représente —O—, —S— ou —$NR^5$—,

X' représente —O— ou —$NR^5$—,

K représente une liaison directe, un radical alkylène en $C_1$-$C_6$ ou un radical de formule :

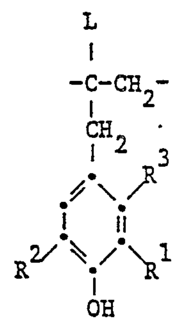

$R^1$ représente l'hydrogène, un alkyle en $C_1$-$C_8$, un cycloalkyle en $C_5$-$C_8$, un phénylalkyle en $C_7$-$C_9$, un phényle ou un alkylphényl en $C_7$-$C_{10}$,

$R^2$ représente un alkyle en $C_1$-$C_8$, un cycloalkyle en $C_5$-$C_8$, un phénylalkyle en $C_7$-$C_9$, un phényle ou un alkylphényle en $C_7$-$C_{10}$,

$R^3$ représente l'hydrogène ou un méthyle,

$R^4$ représente l'hydrogène, un alkyle en $C_1$-$C_{12}$, un cycloalkyle en $C_5$-$C_8$, un phényle ou un benzyle,

$R^5$ représente l'hydrogène, un alkyle en $C_1$-$C_{12}$, un cycloalkyle en $C_5$-$C_{12}$, un allyle ou un benzyle et

L représente —CN, —$COCH_3$ ou —$SO_2CH_3$,

soit

b) un radical répondant à l'une des formules VII à XV :

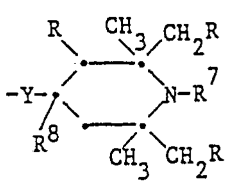

(VII)

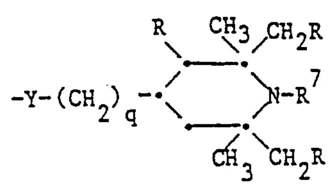

(VIII)

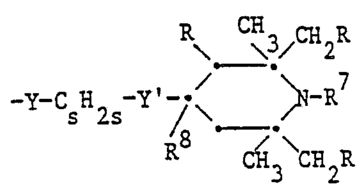

(IX)

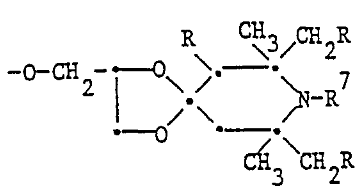

(X)

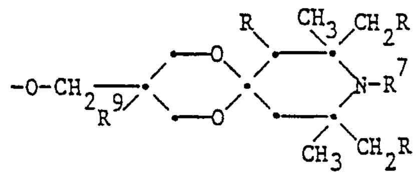

(XI)

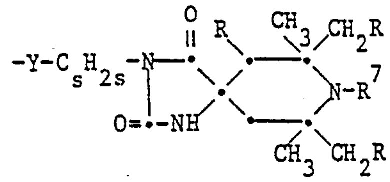

(XII)

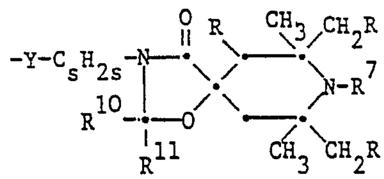

(XIII)

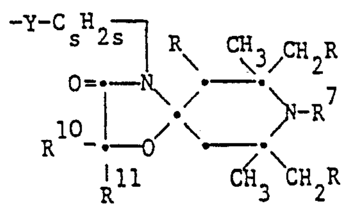

(XIV)

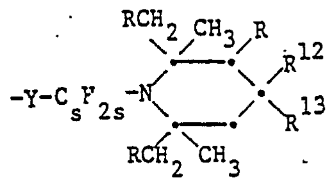

(XV)

dans lesquelles :

Y et Y' représentent chacun, indépendamment l'un de l'autre, —O— ou un radical —N($R^6$)— dans lequel

$R^6$ représente l'hydrogène, un alkyle en $C_1$-$C_{18}$, un alcényle en $C_3$-$C_6$, un cycloalkyle en $C_5$-$C_{12}$, un phénylalkyle en $C_7$-$C_{12}$, un alcoxyalkyle en $C_3$-$C_{12}$, un dialkylamino-alkyle en $C_4$-$C_{12}$, un radical —$C_sH_{2s}$—X'—CO—$R^{14}$, un radical :

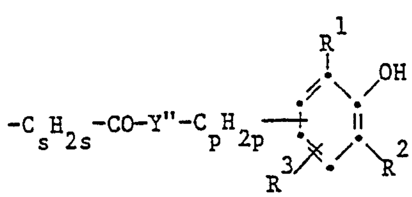

ou un radical de formule XVI :

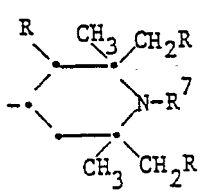

(XVI)

R représente l'hydrogène ou un méthyle,

$R^7$ représente un hydroxy, un alkyle en $C_1$-$C_{12}$, un alcényle en $C_3$-$C_6$, un alcynyle en $C_3$ ou $C_4$, un phénylalkyle en $C_7$-$C_{12}$, un glycidyle, un alkyle en $C_1$-$C_4$ porteur d'un halogène ou d'un radical —CN,

—$COOR^{15}$ ou —$CON(R^{16})$ $(R^{17})$, ou un radical —CO—$R^{14}$, —CO—$OR^{15}$, —CO—$N(R^{16})$ $(R^{17})$, —$CH_2$—$CH(R^{18})$ $OR^{19}$, —SO—$R^{20}$, —$SO_2$—$R^{20}$, —$CR^{15}$ ou —OOC—$R^{14}$,

$R^8$ représente l'hydrogène ou un radical —CN, —$COOR^{15}$, —$CONH_2$ ou —$CON(R^{16})$ $(R^{17})$,

$R^9$ représente un méthyle ou un éthyle,

$R^{10}$ représente l'hydrogène, un alkyle en $C_1$-$C_{12}$, un cycloalkyle en $C_5$-$C_8$ ou un benzyle et

$R^{11}$ un alkyle en $C_1$-$C_{12}$, un cycloalkyle en $C_5$-$C_8$ ou un phényle, ou

$R^{10}$ et $R^{11}$ forment ensemble, et avec l'atome de carbone auxquels ils sont liés, un noyau d'alkylcycloalcane ou de cycloalcane en $C_5$-$C_{12}$,

$R^{12}$ représente l'hydrogène ou un radical —$OR^{21}$, —OOC—$R^{14}$ ou —$N(R^{22})$—CO—$R^{14}$ et

$R^{13}$ l'hydrogène ou un radical —CN, —$COOR^{15}$, —$CONH_2$ ou —$CON(R^{16})$ $(R^{17})$ ou

$R^{12}$ et $R^{13}$ forment ensemble un radical répondant à l'une des formules :

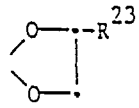

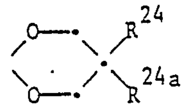

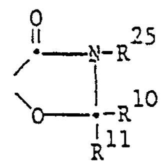

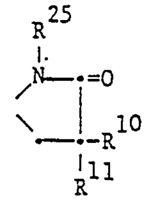

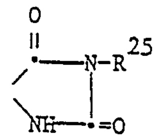

q est égal à 1 ou à 2,

s représente un nombre entier de 2 à 6,

Y" représente —O— ou un radical —$NR^{5a}$— dans lequel $R^{5a}$ a la signification de $R^5$ ou est un radical de formule XVI,

$R^{14}$ représente l'hydrogène, un alkyle en $C_1$-$C_{12}$, un alcényle en $C_2$-$C_6$, un chlorométhyle, un cycloalkyle en $C_5$-$C_{12}$, un phénylalkyle en $C_7$-$C_{12}$, un phényle, un alkylphényle en $C_7$-$C_{10}$ ou un radical phényle, phénylméthyle ou phényléthyle qui porte un ou deux radicaux alkyles en $C_1$-$C_4$ et un radical hydroxy,

$R^{15}$ représente un alkyle en $C_1$-$C_{12}$, un allyle, un benzyle ou un cyclohexyle,

$R^{16}$ représente un alkyle en $C_1$-$C_{12}$, un allyle, un cyclohexyle, un benzyle ou un phényle et

$R^{17}$ l'hydrogène, un alkyle en $C_1$-$C_8$ ou un allyle, ou

$R^{16}$ et $R^{17}$ forment ensemble, et avec l'atome d'azote, un hétérocycle à 5 ou 6 maillons,

$R^{18}$ représente l'hydrogène, un alkyle en $C_1$-$C_4$, un alcoxyméthyle en $C_2$-$C_{13}$, un phényle ou un phénoxyméthyle,

$R^{19}$ représente l'hydrogène, un alkyle en $C_1$-$C_{12}$, un radical —CO—$R^{14}$ ou un radical —CO—$N(R^{16})$ $(R^{17})$,

$R^{20}$ représente un alkyle en $C_1$-$C_{12}$, un phényle ou un alkylaryle en $C_7$-$C_{22}$,

$R^{21}$ représente l'hydrogène, un alkyle en $C_1$-$C_{12}$, un allyle ou un benzyle,

$R^{22}$ représente l'hydrogène, un alkyle en $C_1$-$C_{12}$, un cycloalkyle en $C_5$-$C_{12}$ ou un benzyle,

$R^{23}$ représente l'hydrogène, un méthyle ou un éthyle,

$R^{24}$ et $R^{24a}$ représentent chacun, indépendamment l'un de l'autre, H ou un alkyle en $C_1$-$C_4$ et

$R^{25}$ représente l'hydrogène, un alkyle en $C_1$-$C_{12}$, un alcoxyalkyle en $C_3$-$C_{12}$, un cycloalkyle en $C_5$-$C_{12}$, un allyle ou un benzyle,

soit

c) l'un des radicaux —$OR^{26}$, —$SR^{27}$, —P(O) $(OR^{28})_2$ ou —$N(R^{29})$ $(R^{30})$

dans lesquels :

$R^{26}$ représente l'hydrogène, un alkyle en $C_1$-$C_{18}$, un alcényle en $C_3$-$C_6$, un alcoxyalkyle en $C_3$-$C_{12}$, un dialkylamino-alkyle en $C_4$-$C_{12}$, un phényle, un alkylaryle en $C_7$-$C_{22}$, un phénylalkyle en $C_7$-$C_9$ ou un cycloalkyle en $C_5$-$C_{12}$,

$R^{27}$ représente un alkyle en $C_1$-$C_{18}$, un cycloalkyle en $C_5$-$C_{12}$, un phényle, un alkylphényle en $C_7$-$C_{12}$ ou un phénylalkyle en $C_7$-$C_9$, ou représente un radical —$CH_2COOR^{31}$ ou un radical —$CH_2CH_2COOR^{31}$ dans lesquels $R^{31}$ désigne l'hydrogène ou un alkyle en $C_1$-$C_{12}$,

$R^{28}$ représente un alkyle en $C_1$-$C_{12}$, un phényle, un tolyle, un benzyle ou un radical de formule XVI,

$R^{29}$ et $R^{30}$ représentent chacun, indépendamment l'un de l'autre, l'hydrogène, un alkyle en $C_1$-$C_{12}$, un alkyle en $C_2$-$C_4$ porteur d'un alcoxy en $C_1$-$C_4$, d'un radical $R^{14}$—COO— ou d'un radical dialkylamino en $C_2$-$C_8$, un cycloalkyle en $C_5$-$C_{12}$, un alcényle en $C_3$-$C_6$ ou un phénylalkyle en $C_7$-$C_9$ ou

$R^{29}$ et $R^{30}$ forment ensemble, et avec l'atome d'azote auxquels ils sont liés, un hétérocycle à 5 ou 6 maillons,

et

$R^{III}$ peut également représenter un radical répondant à la formule XVII :

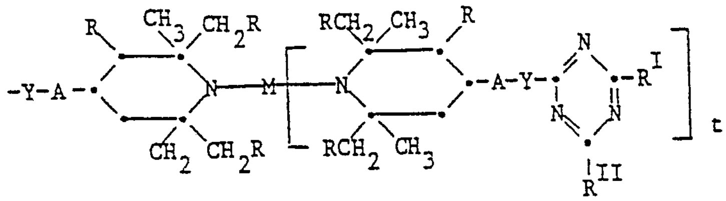

(XVII)

dans laquelle :

t représente un nombre égal à 1, à 2 ou à 3,

A représente une liaison directe, un radical $-(CH_2)_q-$ ou un radical $-Y'-C_sH_{2s}-$ et

M représente, dans le cas où t est égal à 1, un radical bivalent, en l'espèce un alkylène en $C_2$-$C_{12}$, un alcénylène en $C_4$ ou $C_8$, un xylylène ou un radical répondant à l'une des formules :

$-CH_2-C\equiv C-CH_2$ 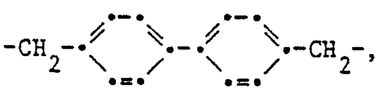 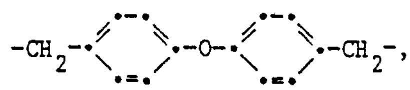

$-CH_2-COO-B-OOC-CH_2-$, $-CH_2-CH(OH)-CH_2-$, $-CH_2CH(OH)CH_2-D-CH_2CH(OH)CH_2-$, $-CH_2-CH(R^{18})-OOC-R^{33}-COO-CH(R^{18})-CH_2-$ et $-CO-NH-G-NH-CO-$

dans lesquelles B représente un alkylène en $C_2$-$C_{12}$, un oxaalkylène en $C_4$-$C_8$ ou un cyclohexylène, D représente un radical bivalent qui est un radical $-O-R^{32}-O-$ (dans lequel $R^{32}$ désigne un alkylène en $C_2$-$C_{12}$, un cycloalkylène en $C_6$-$C_{12}$, un arylène en $C_6$-$C_{12}$ ou un radical -phénylène-Z-phénylène- et Z désigne $-O-$, $-CH_2-$,

$$>C(CH_3)_2$$

ou $-SO_2-$) ou un radical $-OOC-R^{33}-COO-$, $R^{33}$ représente une liaison directe, un alkylène en $C_1$-$C_{12}$, un alcénylène en $C_2$-$C_6$, un cycloalkylène en $C_6$-$C_{12}$, un cycloalcénylène en $C_6$-$C_{12}$ ou un arylène en $C_6$-$C_{12}$, et G représente un radical bivalent aliphatique, cycloaliphatique, aromatique ou aromatique-aliphatique qui contient de 6 à 15 atomes de carbone,

M représente, dans le cas où t est égal à 2, un radical trivalent répondant à l'une des formules :

$$T-[-CH_2CH(OH)\ CH_2-]-_3 \quad \text{et} \quad R^{34}-[-COO-CH(R^{18})-CH_2-]-_3$$

dans lesquelles T représente un radical trivalent de formule :

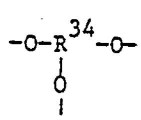 ou 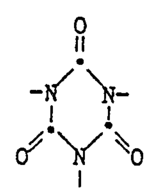

et $R^{34}$ représente un radical hydrocarboné aliphatique trivalent contenant de 3 à 10 atomes de carbone ou un radical hydrocarboné aromatique trivalent contenant de 6 à 10 atomes de carbone, et

M représente, dans le cas où t est égal à 3, un radical quadrivalent répondant à l'une des formules :

$$Q-[-CH_2CH(OH)\ CH_2-]-_4 \quad \text{et} \quad R^{35}-[-COO-CH(R^{18})-CH_2-]-_4$$

dans lesquelles

Q représente un radical répondant à l'une des formules :

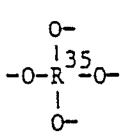 ou 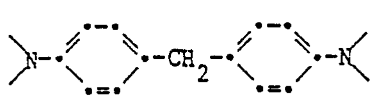 et

et $R^{35}$ représente un radical hydrocarboné aliphatique quadrivalent qui contient de 4 à 10 atomes de carbone ou un radical hydrocarboné aromatique quadrivalent qui contient de 6 à 12 atomes de carbone,

$R^{IV}$ représente le radical, devalence m, d'une polyamine ou d'un polyol,

m représente un nombre entier de 2 à 6,

$R^{V}$ représente un radical bivalent

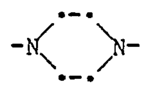

ou un radical —Y—$R^{36}$—Y— dans lequel $R^{36}$ représente un alkylène en $C_2$-$C_{12}$, un cycloalkylène en $C_5$-$C_{12}$, un aralkylène en $C_8$-$C_{12}$, un arylène en $C_6$-$C_{12}$, un alkylène en $C_4$-$C_8$ interrompu par O ou $NR^5$, ou un radical répondant à l'une des formules :

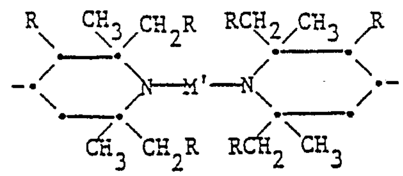

et

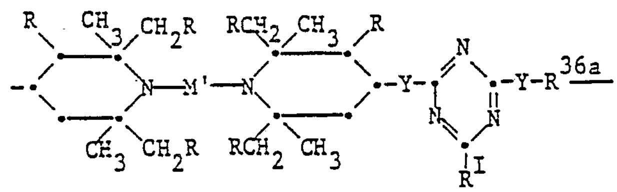

dans lesquelles :

M' représente un radical bivalent de formule M et

$R^{36a}$ représente un alkylène en $C_2$-$C_{12}$, un cycloalkylène en $C_5$-$C_{12}$, un aralkylène en $C_8$-$C_{12}$, un arylène en $C_6$-$C_{12}$ ou un alkylène en $C_4$-$C_8$ interrompu par O ou $NR^5$,

n représente un nombre de 2 à 50 et E et E' représentent des radicaux terminaux,

et dans lesquelles au moins un des radicaux $R^{I}$, $R^{II}$ et $R^{III}$ contient un radical de phénol à empêchement stérique et au moins un des radicaux $R^{I}$, $R^{II}$, $R^{III}$, $R^{IV}$ et $R^{V}$ contient un radical de polyalkyl-pipéridine.

2. Matière pour enregistrement photographique en couleurs selon la revendication 1, qui contient, comme stabilisant, un composé de formule I, II ou III dont la molécule renferme au moins un radical de phénol à empêchement stérique et au moins un radical de tétraméthyl-2,2,6,6 pipéridine substituée à l'azote.

3. Matière pour enregistrement photographique en couleurs selon la revendication 1, qui contient, comme stabilisant, un composé de formule I dans lequel $R^{I}$, $R^{II}$ et $R^{III}$ représentent chacun, indépendamment les uns des autres :

a) un radical répondant à la formule IVa :

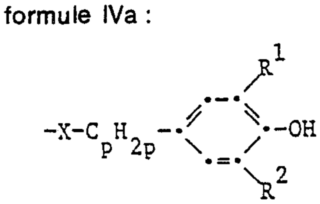

(IVa)

dans laquelle p représente un nombre entier de 1 à 4, et X, $R^1$ et $R^2$ ont les significations données à la revendication 1,

b) un radical répondant à l'une des formules VIIa et XVa :

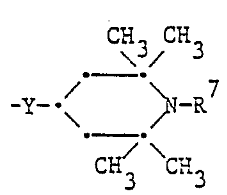

(VIIa)

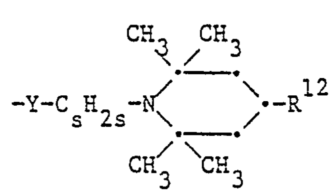

(XVa)

dans lesquelles Y représente —O— ou —$NR^6$—, et $R^6$, $R^7$, $R^{12}$ et s ont les significations données à la revendication 1, ou

c) l'un des radicaux $—OR^{26}$, $—SR^{27}$, $—P(O)(OR^{28})_2$ ou $—N(R^{29})(R^{30})$ dans lesquels $R^{26}$, $R^{27}$, $R^{28}$, $R^{29}$ et $R^{30}$ ont les significations données à la revendication 1,
et dans lequel au moins un des radicaux $R^I$, $R^{II}$ et $R^{III}$ contient un radical de phénol à empêchement stérique et au moins un des radicaux $R^I$, $R^{II}$ et $R^{III}$ contient un radical de tétraméthyl-2,2,6,6 pipéridine substitué à l'azote.

4. Matière pour enregistrement photographique en couleurs selon la revendication 3, qui contient, comme stabilisant, un composé de formule I dans lequel $R^I$, $R^{II}$ et $R^{III}$ représentent chacun, indépendamment les uns des autres, soit :

a) un radical de formule IVa dans lequel $R^1$ représente l'hydrogène ou un alkyle en $C_1$-$C_4$, $R^2$ représente un alkyle en $C_1$-$C_4$, et X et p ont les significations données à la revendication 3, soit

b) un radical répondant à l'une des formules VIIa et XVa dans lesquelles Y représente —O— ou $—NR^6—$, et $R^6$ représente l'hydrogène, un alkyle en $C_1$-$C_8$, un allyle, un cyclohexyle, un benzyle ou un radical $—C_sH_{2s}—X'—CO—R^{14}$ dans lequel s est égal à 2 ou à 3, X' représente —O— et $R^{14}$ représente un radical phényle, phénylméthyle ou phényléthyle porteur d'un ou deux alkyles en $C_1$-$C_4$ et d'un hydroxy, $R^7$ représente un radical méthyle, allyle, benzyle, hydroxy-2 éthyle, acétyle ou acryloyle ou un radical $—CON(R^{16})(R^{17})$ dans lequel $R^{16}$ désigne un alkyle en $C_1$-$C_8$, un cyclohexyle ou un phényle et $R^{17}$ l'hydrogène ou un alkyle en $C_1$-$C_8$, et $R^{12}$ représente l'hydrogène, soit

c) l'un des radicaux $—OR^{26}$, $—SR^{27}$ et $—N(R^{29})(R^{30})$ dans lesquels $R^{26}$ représente un alkyle en $C_1$-$C_{12}$, un phényle, un alkylaryle en $C_7$-$C_{22}$, un benzyle ou un cyclohexyle, $R^{27}$ un alkyle en $C_1$-$C_{12}$, un cyclohexyle, un phényle, un radical $—CH_2COO$(alkyl en $C_1$-$C_{12}$) ou un radical $—CH_2CH_2COO$ (alkyl en $C_1$-$C_{12}$), et $R^{29}$ et $R^{30}$ représentent chacun l'hydrogène, un alkyle en $C_1$-$C_{12}$, un benzyle ou un allyle.

5. Matière pour enregistrement photographique en couleurs selon la revendication 1, qui contient, comme stabilisant, un composé de formule II dans lequel m est égal à 2 et $R^{IV}$ représente un radical bivalent répondant à l'une des formules :

$$-N(R^6)-R^{37}-N(R^6)-$$ et 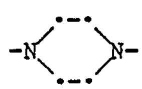

où $R^{37}$ représente un alkylène en $C_2$-$C_{12}$, un alkylène en $C_4$-$C_8$ interrompu par —O— ou $—NR^5—$, un arylène en $C_6$-$C_{15}$, un aralkylène en $C_8$-$C_{12}$ ou un cycloalkylène en $C_5$-$C_{12}$, et $R^5$ et $R^6$ ont les significations données à la revendication 1.

6. Matière pour enregistrement photographique en couleurs selon la revendication 1, qui contient, comme stabilisant, un composé de formule II dans lequel m est égal à 3 et $R^{IV}$ représente un radical trivalent de formule :

$$-N(R^6)-(CH_2)_a-N(R^6)-(CH_2)_a-N(R^6)-$$

où m est égal à 4 et $R^{IV}$ représente un radical quadrivalent de formule :

$$-N(R^6)-(CH_2)_a-N(R^6)-(CH_2)_b-N(R^6)-(CH_2)_a-N(R^6)-$$

formules dans lesquelles a est égal à 2 ou à 3, b désigne un nombre de 2 à 12 et $R^6$ a la signification qui lui a été donnée à la revendication 1.

7. Matière pour enregistrement photographique en couleurs selon la revendication 1, qui contient, comme stabilisant, un composé de formule III dans lequel $R^V$ représente un radical bivalent $—N(R^6)—R^{36a}—N(R^6)—$ dans lequel $R^{36a}$ et $R^6$ ont les significations données à la revendication 1.

8. Matière pour enregistrement photographique en couleurs selon la revendication 7, caractérisée en ce que, dans le composé de formule III, le radical terminal E est essentiellement l'hydrogène et le radical terminal E' est essentiellement un radical $—N(R^6)—R^{36a}—NHR^6$.

9. Matière pour enregistrement photographique en couleurs selon la revendication 7, qui contient, comme stabilisant, un composé de formule III dans lequel $R^I$ représente un radical :

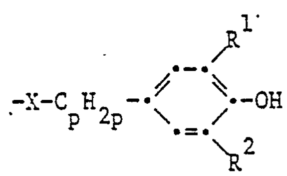

et $R^5$ représente un radical :

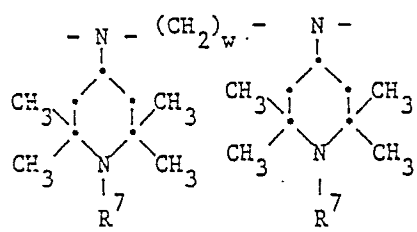

radicaux dans lesquels w désigne un nombre de 2 à 6, et $R^1$, $R^2$, X, p et $R^7$ ont les significations données à la revendication 1.

10. Matière pour enregistrement photographique en couleurs selon la revendication 9, qui contient, comme stabilisant, un composé de formule III dans lequel $R^{I}$ représente un radical :

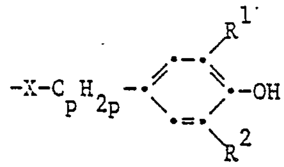

et $R^{V}$ représente un radical :

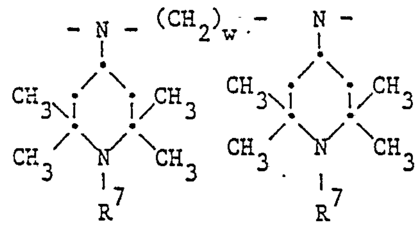

radicaux dans lesquels :

p désigne un nombre de 1 à 4,

X représente —O—, —S—, —NH— ou —N(alkyl en $C_1$-$C_4$),

$R^1$ représente l'hydrogène ou un alkyle en $C_1$-$C_4$,

$R^2$ représente un alkyle en $C_1$-$C_4$,

w désigne un nombre de 2 à 6 et

$R^7$ représente un radical méthyle, allyle, benzyle, hydroxy-2 éthyle, acétyle ou acryloyle ou un radical —CON($R^{16}$) ($R^{17}$) dans lequel $R^{16}$ représente un alkyle en $C_1$-$C_8$, un cyclohexyle ou un phényle et $R^{17}$ l'hydrogène ou un alkyle en $C_1$-$C_8$.

11. Matière pour enregistrement photographique en couleurs selon la revendication 1, caractérisée en ce qu'elle contient, en plus d'un stabilisant de formule I, II ou III, un stabilisant à la lumière pris dans l'ensemble des absorbeurs de rayons ultra-violets.

12. Matière pour enregistrement photographique en couleurs selon la revendication 1, caractérisée en ce qu'elle contient, par mètre carré, de 1 à 2 000 mg d'un composé de formule I, II ou III, de préférence de 100 à 800 mg.

13. Application de composés s-triaziniques de formule I, II ou III, tels que définis à la revendication 1, comme stabilisants pour des matières destinées à l'enregistrement photographique en couleurs.